(19) Europäisches Patentamt
European Patent Office
Office européen des brevets

(11) EP 4 063 379 A1

(12) **EUROPEAN PATENT APPLICATION**
published in accordance with Art. 153(4) EPC

(43) Date of publication:
**28.09.2022 Bulletin 2022/39**

(21) Application number: **20895099.8**

(22) Date of filing: **01.12.2020**

(51) International Patent Classification (IPC):
***C07K 7/08*** (2006.01) ***C07K 14/52*** (2006.01)
***C07K 14/47*** (2006.01) ***C12N 9/02*** (2006.01)
***C12N 9/18*** (2006.01) ***C12N 9/90*** (2006.01)

(52) Cooperative Patent Classification (CPC):
**C07K 7/08; C07K 14/47; C07K 14/52; C12N 9/0004; C12N 9/18; C12N 9/90**

(86) International application number:
**PCT/KR2020/017413**

(87) International publication number:
**WO 2021/112540 (10.06.2021 Gazette 2021/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2019 KR 20190158447**

(71) Applicant: **LG Chem, Ltd.**
**Seoul 07336 (KR)**

(72) Inventors:
- **MIN, Byeong Eun**
  **Seoul 07796 (KR)**
- **KIM, Yeonchul**
  **Seoul 07796 (KR)**
- **PARK, Young Sam**
  **Seoul 07796 (KR)**
- **JUNG, Saem**
  **Seoul 07796 (KR)**

(74) Representative: **Hoffmann Eitle**
**Patent- und Rechtsanwälte PartmbB**
**Arabellastraße 30**
**81925 München (DE)**

# (54) CHO CELL-DERIVED PROTEIN SECRETORY FACTOR AND EXPRESSION VECTOR COMPRISING SAME

(57) The present invention relates to a CHO cell-derived protein secretory factor, an expression cassette in which a nucleic acid sequence encoding the protein secretory factor; and a gene encoding a target protein are operably linked, an expression vector comprising the expression cassette, a transformed cell into which the expression vector is introduced, and a method for producing a target protein using the transformed cell.

[Fig. 2]

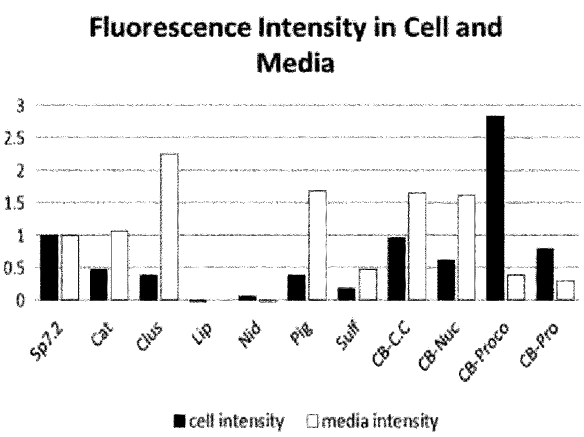




EP 4 063 379 A1

## Description

### [Technical Field]

The present invention relates to a CHO cell-derived protein secretory factor, an expression cassette in which a nucleic acid sequence encoding the protein secretory factor; and a gene encoding a target protein are operably linked, an expression vector including the expression cassette, a transformed cell into which the expression vector is introduced, and a method for producing a target protein using the transformed cell.

### [Background Art]

Recombinant proteins can be produced on a large-scale using microbial or animal cell systems through genetic recombination technology for useful protein components that are difficult to obtain *in vivo*. Recombinant proteins can be regulated such that they can be expressed in cells or secreted outside the cells. However, the intracellular expression has a disadvantage in that the proteins are often accumulated as an insoluble mass, and that productivity is lowered due to difficulty in separation and purification. On the other hand, soluble proteins with correct protein folding can be easily obtained through extracellular secretion. Thus, for more suitable extracellular secretion in terms of protein production yield and quality control, an optimized recombinant protein expression system is important.

In order to produce a recombinant protein, components such as a host cell, a gene of interest, an expression vector, a selective marker, a promoter, and a signal peptide sequence are essentially required. The quality and productivity of the recombinant protein vary depending on the selection of these components.

In the case of the signal peptide, it is located at the N-terminal region of the recombinant protein to be produced and thus is involved in the expression level of the recombinant protein, and is a component that allows the extracellular secretion. Depending on which signal peptide is used, a difference in expression level can be observed, and the signal peptide sequence may remain at the N-terminus of the protein due to the mis-cleavage of the signal peptide, affecting the quality of the recombinant protein.

Therefore, it is important to select a signal peptide that does not cause mis-cleavage and can induce high expression.

Meanwhile, conventional signal peptides mostly use human-derived signal peptides, and CHO cells are mainly used as expression host cells. Signal peptides between host cells may be used in combination, but may cause problems in terms of quality.

Under these circumstances, the present inventors have made extensive efforts to increase the expression level in CHO cells and solve the mis-cleavage problem. As a result, they have developed a novel signal peptide, which is a polypeptide consisting of 17 to 31 amino acid sequences derived from CHO cells, and have completed the present invention by confirming that the signal peptide can be significantly increased in terms of expression and cleaved 100% at a cleavage site to prevent mis-cleavage.

### [Disclosure]

### [Technical Problem]

An object of the present invention is to provide a protein secretory factor consisting of an amino acid sequence of SEQ ID NO: 1, SEQ ID NO: 2, or SEQ ID NO: 3.

Another object of the present invention is to provide an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked.

Still another object of the present invention is to provide an expression vector for secreting a target protein, including an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked.

Still another object of the present invention is to provide a transformed cell in which the expression vector is introduced into a host cell.

Still another object of the present invention is to provide a method for producing a target protein, including:

i) culturing a transformed cell including an expression vector for secreting a target protein, which includes an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked; and

ii) recovering the target protein from the culture medium or culture supernatant of the cultured cells.

**[Technical Solution]**

Hereinbelow, the present invention will be described in detail. Meanwhile, each of the explanations and exemplary embodiments disclosed herein can be applied to other explanations and exemplary embodiments. That is, all combinations of various factors disclosed herein belong to the scope of the present invention. Furthermore, the scope of the present invention should not be limited by the specific disclosure provided hereinbelow.

Additionally, those of ordinary skill in the art may be able to recognize or confirm, using only conventional experimentation, many equivalents to the particular aspects of the invention described herein. Furthermore, it is also intended that these equivalents be included in the present invention.

In order to achieve the objects above, one aspect of the present invention provides a novel protein secretory factor derived from CHO cells. Specifically, the present invention provides a protein secretory factor consisting of an amino acid sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 10. More specifically, the protein secretory factor may consist of an amino acid sequence of SEQ ID NO: 1, SEQ ID NO: 2, or SEQ ID NO: 3, but is not limited thereto.

The "protein secretory factor" of the present invention refers to a factor that is linked to a target protein to induce the extracellular secretion of the target protein, and may consist of a polypeptide. The protein secretory factor may promote the secretion of a target protein, that is, an endogenous protein and/or a foreign protein, and in particular, may promote the extracellular secretion of the light and/or heavy chain of an antibody, but is not limited thereto.

The protein secretory factor in the present invention may be interchangeably used with "signal sequence" or "signal peptide (SP)".

The protein secretory factor of the present invention may have an amino acid sequence of SEQ ID NO: 1, SEQ ID NO: 2, or SEQ ID NO: 3, but is not limited thereto. Additionally, the protein secretory factor of the present invention may further include a protein secretory factor consisting of an amino acid sequence of SEQ ID NO: 4, SEQ ID NO: 5, SEQ ID NO: 6, SEQ ID NO: 7, SEQ ID NO: 8, SEQ ID NO: 9, or SEQ ID NO: 10, but is not limited thereto.

In one specific embodiment of the present invention, the protein secretory factor may be derived from CHO cells, but is not limited thereto. As used herein, the term "CHO cell" is a Chinese hamster ovary cell, and may be a host cell for transformation commonly used in the art. In addition, a CHO-cell derived protein secretory factor may be selected to increase the expression level in CHO cells, which are host cells.

In the present invention, the protein secretory factors consisting of the amino acid sequence of SEQ ID NO: 1 may be cathepsin B (Cat), and may be used interchangeably with the Cat secretion sequence in the present invention. The protein secretory factor consisting of the amino acid sequence of SEQ ID NO: 2 may be a C-C motif chemokine (CC), and may be used interchangeably with the CC secretion sequence in the present invention. The protein secretory factor consisting of the amino acid sequence of SEQ ID NO: 3 may be nucleobindin-2 (NUC), and may be used interchangeably with the NUC secretion sequence in the present invention.

Additionally, the protein secretory factor consisting of the amino acid sequence of SEQ ID NO: 4 of the present invention may be clusterin (Clus), and may be used interchangeably with the Clus secretion sequence in the present invention. The protein secretory factor consisting of the amino acid sequence of SEQ ID NO: 5 may be a pigment epithelium-derived factor (Pig), and may be used interchangeably with the Pig secretion sequence in the present invention. The protein secretory factor consisting of the amino acid sequence of SEQ ID NO: 6 may be procollagen C-endopeptidase enhancer 1 (Proco), and may be used interchangeably with the Proco secretion sequence in the present invention. The protein secretory factor consisting of the amino acid sequence of SEQ ID NO: 7 may be sulfhydryl oxidase (Sulf), and may be used interchangeably with the Sulf secretion sequence in the present invention. The protein secretory factor consisting of the amino acid sequence of SEQ ID NO: 8 may be lipoprotein lipase (Lip), and may be used interchangeably with the Lip secretion sequence in the present invention. The protein secretory factor consisting of the amino acid sequence of SEQ ID NO: 9 may be nidogen-1 (Nid), and may be used interchangeably with the Nid secretion sequence in the present invention. The protein secretory factor consisting of the amino acid sequence of SEQ ID NO: 10 may be protein disulfide-isomerase (Pro), and may be used interchangeably with the Pro secretion sequence in the present invention.

The nucleic acid sequence encoding the cathepsin B signal peptide consisting of the amino acid sequence of SEQ ID NO: 1 may be a polynucleotide sequence of SEQ ID NO: 11, the nucleic acid sequence encoding the C-C motif chemokine signal peptide consisting of the amino acid sequence of SEQ ID NO: 2 may be a polynucleotide sequence of SEQ ID NO: 12, and the nucleic acid sequence encoding the nucleobindin-2 signal peptide consisting of the amino acid sequence of SEQ ID NO: 3 may be a polynucleotide sequence of SEQ ID NO: 13.

Additionally, the nucleic acid sequence encoding the clusterin signal peptide consisting of the amino acid sequence of SEQ ID NO: 4 may be a polynucleotide sequence of SEQ ID NO: 14, the nucleic acid sequence encoding the pigment epithelium-derived factor (Pig) signal peptide consisting of the amino acid sequence of SEQ ID NO: 5 may be a polynucleotide sequence of SEQ ID NO: 15, the nucleic acid sequence encoding the procollagen C-endopeptidase enhancer 1 (Proco) signal peptide consisting of the amino acid sequence of SEQ ID NO: 6 may be a polynucleotide sequence of SEQ ID NO: 16, the nucleic acid sequence encoding the sulfhydryl oxidase (Sulf) signal peptide consisting of the amino acid sequence of SEQ ID NO: 7 may be a polynucleotide sequence of SEQ ID NO: 17, the nucleic acid sequence encoding the lipoprotein lipase (Lip) signal peptide consisting of the amino acid sequence of SEQ ID NO: 8 may be a polynucleotide sequence of SEQ ID NO: 18, the nucleic acid sequence encoding the nidogen-1 (Nid) signal peptide consisting of the amino acid sequence of SEQ ID NO: 9 may be a polynucleotide sequence of SEQ ID NO: 19, and the nucleic acid sequence encoding the protein disulfide-isomerase (Pro) signal peptide consisting of the amino acid sequence of SEQ ID NO: 10 may be a polynucleotide sequence of SEQ ID NO: 20.

Although the protein secretory factor of the present invention is described as "a secretory factor consisting of a specific amino acid sequence", it is apparent that as long as the secretory factor has an activity identical or corresponding to that of a secretory factor consisting of an amino acid sequence of the corresponding sequence number, it does not exclude a mutation that may occur by a meaningless sequence addition upstream or downstream of the amino acid sequence, a mutation that may occur naturally, or a silent mutation thereof. Even when the sequence addition or mutation is present, it falls within the scope of the present invention.

For example, as long as the secretory factor can function as a signal peptide identically or correspondingly to the nucleic acid molecules consisting of the polynucleotides, nucleic acid sequences showing a homology and/or identity of 85% or more, specifically 90% or more, more specifically 95% or more, even more specifically 98% or more, or even more specifically 99% or more to the sequence above can also be included in the present invention without limitation. Additionally, it is obvious that a nucleic acid sequence with deletion, modification, substitution, or addition in part of the sequence also can be included in the scope of the present invention, as long as the nucleic acid sequence has such homology.

As used herein, the term "homology" or "identity" refers to a degree of relevance between two given amino acid sequences or nucleic acid sequences, and may be expressed as a percentage. The terms "homology" and "identity" may often be used interchangeably with each other.

The sequence homology or identity of conserved polynucleotide or polypeptide sequences may be determined by standard alignment algorithms and can be used with a default gap penalty established by the program being used. Substantially, homologous or identical sequences are generally expected to hybridize to all or at least about 50%, 60%, 70%, 80%, or 90% or more of the entire length of the sequences under moderate or high stringent conditions. Polynucleotides that contain degenerate codons instead of codons in the hybridizing polypeptides are also considered.

Whether any two polynucleotide or polypeptide sequences have a homology, similarity, or identity may be determined by a known computer algorithm such as the "FASTA" program (Pearson et al., (1988) [Proc. Natl. Acad. Sci. USA 85]: 2444) using default parameters. Alternatively, it may be determined by the Needleman-Wunsch algorithm (Needleman and Wunsch, 1970, J. Mol. Biol. 48: 443-453), which is performed using the Needleman program of the EMBOSS package (EMBOSS: The European Molecular Biology Open Software Suite, Rice et al., 2000, Trends Genet. 16: 276-277) (preferably, version 5.0.0 or versions thereafter) (GCG program package (Devereux, J., et al., Nucleic Acids Research 12: 387 (1984)), BLASTP, BLASTN, FASTA (Atschul, [S.] [F.,] [ET AL., J MOLEC BIOL 215]: 403 (1990); Guide to Huge Computers, Martin J. Bishop, [ED.,] Academic Press, San Diego, 1994, and [CARILLO ETA/.](1988) SIAM J Applied Math 48: 1073). For example, the homology, similarity, or identity may be determined using BLAST or ClustalW of the National Center for Biotechnology Information (NCBI).

The homology, similarity, or identity of polynucleotides or polypeptides may be determined by comparing sequence information using, for example, the GAP computer program, such as Needleman et al. (1970), J Mol Biol. 48: 443 as disclosed in Smith and Waterman, Adv. Appl. Math (1981) 2:482. In summary, the GAP program defines the homology, similarity, or identity as the value obtained by dividing the number of similarly aligned symbols (i.e. nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps. Therefore, as used herein, the term "homology" or "identity" refers to the relevance between sequences.

Another aspect of the present invention provides an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked

The "protein secretory factor" of the present invention is as described above.

As used herein, the term "target protein" may refer to a protein endogenouslyexpressed in a host cell or a protein expressed by a foreign gene introduced thereinto. The type of target protein is not particularly limited as long as extracellular secretion efficiency is increased by the signal peptide sequence of the present invention.

The target protein may be antibody, antibody fragment (Fab or ScFv), fusion protein, protein scaffold, human growth hormone, serum protein, immunoglobulin, cytokine, α-, β- or γ-interferon, granulocyte-macrophage colony-stimulating factor (GM-CSF), platelet-derived growth factor (PDGF), phospholipase-activating protein (PLAP), insulin, tumor necrosis factor (TNF), growth factor, hormone, calcitonin, calcitonin gene-related peptide (CGRP), enkephalin, somatomedin, erythropoietin, hypothalamic-releasing factor, growth differentiation factor, cell adhesion protein, prolactin, chorionic gonadotropin, tissue plasminogen activator, growth hormone releasing peptide (GHPR), thymic humoral factor (THF), asparaginase, arginase, arginine deaminase, adenosine deaminase, peroxide dismutase, endotoxinase, catalase, chymotrypsin, lipase, uricase, adenosine diphosphatase, tyrosinase, bilirubin oxidase, glucose oxidase, glucodase, galactosidase, glucocerebrosidase, or glucuronidase, and specifically, it may be the heavy chain protein or the light chain protein of an antibody, but is not limited thereto.

As used herein, the term "operably linked" refers to a functional linkage between the above gene sequence, a promoter sequence, and a signal peptide sequence to initiate and mediate the transcription of the nucleic acid sequence encoding the protein secretory factor of the present application and the gene encoding the target protein. The operable linkage may be prepared using a gene recombination technique known in the art, and the site-specific DNA linkage may be prepared using a linking enzyme known in the art, but is not limited thereto

As used herein, the term "expression cassette" refers to a sequence regulating one or more genes and expression thereof, for example, a nucleic acid sequence including any combination of various cis-acting transcription regulating elements. The expression cassette of the present invention may further include various elements, for example, nucleic acid sequences such as a promoter and an enhancer, which are recognized in the art to be necessary for expression regulation, as well as the nucleic acid sequence encoding the protein secretion factor and the target protein. The sequence regulating the expression of a gene, that is, the sequence regulating the transcription of a gene and the expression of the transcription product thereof, is generally referred to as a "regulatory unit". Most of the regulatory unit is located upstream of a coding sequence of a target gene such that it is operably linked thereto. In addition, the expression cassette may include a 3' non-transcriptional region including a poly-adenylation site at a 3' terminal.

The expression cassette of the present invention may be a combination of polynucleotides, which allows the extracellular secretion and expression of target proteins in a host cell, by operably linking the nucleic acid sequence encoding the protein secretory factor consisting of the amino acid sequence of SEQ ID NO: 1, SEQ ID NO: 2, SEQ ID NO: 3, SEQ ID NO: 4, or SEQ ID NO: 5; and the gene encoding the target protein.

Still another aspect of the present invention provides an expression cassette in which a gene encoding a target protein is operably linked to a nucleic acid sequence encoding a protein secretory factor consisting of an amino acid sequence of SEQ ID NO: 11 to SEQ ID NO: 20, and.

The "protein secretory factor", "target protein", "operably linked", and "expression cassette" of the present invention are as described above.

In the present invention, the protein secretory factor encoded by the polynucleotide sequence of SEQ ID NO: 11 may be cathepsin B (Cat), the protein secretory factor encoded by the polynucleotide sequence of SEQ ID NO: 12 may be a C-C motif chemokine (CC), the protein secretory factor encoded by the polynucleotide sequence of SEQ ID NO: 13 may be nucleobindin-2 (Nuc), the protein secretory factor encoded by the polynucleotide sequence of SEQ ID NO: 14 may be clusterin (Clus), the protein secretory factor encoded by the polynucleotide sequence of SEQ ID NO: 15 may be a pigment epithelium-derived factor (Pig), the protein secretory factor encoded by the polynucleotide sequence of SEQ ID NO: 16 may be procollagen C-endopeptidase enhancer 1 (Proco), the protein secretory factor encoded by the polynucleotide sequence of SEQ ID NO: 17 may be sulfhydryl oxidase (Sulf), the protein secretory factor encoded by the polynucleotide sequence of SEQ ID NO: 18 may be lipoprotein lipase (Lip), the protein secretory factor encoded by the polynucleotide sequence of SEQ ID NO: 19 may be nidogen-1 (Nid), and the protein secretory factor encoded by the polynucleotide sequence of SEQ ID NO: 20 may be protein disulfide-isomerase (Pro).

Still another aspect of the present invention provides an expression vector for secreting a target protein, including an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked.

The "protein secretory factor", "target protein", "operably linked", and "expression cassette" of the present invention are as described above.

As used herein, the term "expression vector for secreting a target protein" refers to an expression vector, in which the protein secretory factor and the gene encoding the target protein are operably linked to induce the extracellular secretion of the target protein when the vector is introduced into a host cell and expressed therein.

As used herein, the term "expression vector" generally refers to a double-stranded DNA fragment as a carrier into which a target DNA fragment encoding a target protein is inserted. The expression vector used in expressing a protein in the art may be used without limitation. Once the expression vector is in a host cell, the expression vector can be replicated regardless of a host chromosomal DNA, and the inserted target DNA can be expressed. In order to increase the expression level of a transfected gene in a host cell, the transfected gene must be operably linked to transcription and translation control sequences which are operated in a selected expression host cell.

The expression vector used in the present invention is not particularly limited as long as it can be replicated in a host cell, and any vector known in the art may be used. Examples of conventionally used vectors may include natural or recombinant plasmids, cosmids, viruses, and bacteriophages. For example, as a phage vector or cosmid vector, pWE15, M13, λMBL3, λMBL4, λIXII, λASHII, λAPII, λt10, λt11, Charon4A, and Charon21A, *etc.* may be used, and as a plasmid vector, those based on pBR, pUC, pBluescriptII, pGEM, pTZ, pCL, pET, *etc.* may be used. Specifically, the vector may be those based on pTZ, but is not limited thereto.

In one specific embodiment of the present invention, an expression vector for secreting a target protein was prepared by operably linking the nucleic acid sequence encoding the protein secretory factor consisting of the amino acid sequence of SEQ ID NO: 1, SEQ ID NO: 2, or SEQ ID NO: 3 and the gene encoding the target protein, based on the pTz-D1G1 vector (a variant including the promoter of Korean Patent No. 10-1038126) (Example 4).

The expression vector may further include a nucleic acid sequence encoding a protein secretory factor consisting of an amino acid sequence of SEQ ID NO: 6, SEQ ID NO: 7, SEQ ID NO: 8, SEQ ID NO: 9, or SEQ ID NO: 10.

Still another aspect of the present invention provides a transformed cell in which the expression vector is introduced into a host cell.

The "expression vector" of the present invention is as described above.

As used herein, the term "transformation" refers to a process of introducing a vector including a polynucleotide encoding a target polypeptide into a host cell, thereby allowing the expression of the protein encoded by the polynucleotide in the host cell.

As long as the transformed polynucleotide can be expressed in a host cell, it does not matter whether it is inserted into the chromosome of a host cell and located therein, or located outside the chromosome, and both cases may be included. Additionally, the polynucleotide includes DNA and RNA which encode the target polypeptide. The polynucleotide may be introduced in any form as long as it can be introduced into a host cell and expressed therein. For example, the polynucleotide may be introduced into a host cell in the form of an expression cassette, which is a gene construct including all elements necessary for self-expression. The expression cassette may conventionally include a promoter operably linked to the polynucleotide, a transcription termination signal, a ribosome-binding domain, and a translation termination signal.

The method of transforming the vector of the present invention includes any method of introducing a nucleic acid into a cell, and can be performed by selecting an appropriate standard technique as known in the art depending on the host cell. For example, the transformation may be carried out via particle bombardment, electroporation, calcium phosphate ($CaPO_4$) precipitation, calcium chloride ($CaCl_2$) precipitation, microinjection, a polyethylene glycol (PEG) technique, a DEAE-dextran technique, a cationic liposome technique, a lithium acetate-DMSO technique, but the method is not limited thereto.

As used herein, the term "host cell" refers to a eukaryotic cell into which a nucleic acid molecule having the activity of the protein secretory factor of the present invention is introduced and can act as a signal peptide. The host cell may include, for example, generally known eukaryotic hosts such as yeasts; insect cells such as *Spodoptera frupperda;* and animal cells such as CHO, COS1, COS7, BSC1, BSC40, and BMT10, but is not limited thereto.

In the present invention, examples of the host cell may be an animal host cell, and specifically, it may be a Chinese hamster ovary cell (CHO cell), but is not limited thereto.

In one specific embodiment of the present invention, the Chinese Hamster Ovary (CHO) cell, which is widely used in the production of recombinant proteins was used as the host cell (Example 4).

As used herein, the term "transformant" refers to a transformed animal cell in which an expression vector including a signal peptide consisting of an amino acid sequence of SEQ ID NO: 1, SEQ ID NO: 2, SEQ ID NO: 3, SEQ ID NO: 4, or SEQ ID NO: 5 and a target protein is introduced into a CHO cell, which is the host cell.

In one specific embodiment of the present invention, it was confirmed that the transformant increased the expression levels of the light and heavy chains of pembrolizumab (i.e., an antibody) which is the target protein (Example 4).

Still another aspect of the present invention provides a method for producing a target protein, including:

i) culturing a transformed cell including an expression vector for secreting a target protein, which includes an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked; and
ii) recovering the target protein from the culture medium or culture supernatant of the cultured cells.

The "protein secretory factor", "target protein", "operably linked", "expression cassette", "expression vector for secreting a target protein", "host cell", and "transformant" of the present invention are as described above.

As used herein, the term "culturing" refers to a process of growing the transformed cells in appropriately, artificially controlled environmental conditions. In the present invention, the method for producing a target protein using the CHO cells as the host cell may be performed using a method widely known in the art. Specifically, the culturing may be performed by a batch process, a fed batch or repeated fed batch process in a continuous manner, but is not limited thereto.

The medium used for the culturing should satisfy the requirements for a specific strain in an appropriate manner. Carbon sources that may be used in the present invention may include sugars and carbohydrates such as glucose, sucrose, lactose, fructose, maltose, starch, and cellulose; oils and fats such as soybean oil, sunflower oil, castor oil, and coconut oil; fatty acids such as palmitic acid, stearic acid, and linoleic acid; alcohols such as ethanol; and organic acids such as gluconic acid, acetic acid, and pyruvic acid, but these are not limited thereto. These substances may be used alone or in a mixture.

Nitrogen sources that may be used in the present invention may include peptone, yeast extract, meat extract, malt extract, corn steep liquor, defatted soybean cake, and urea or inorganic compounds, for example, ammonium sulfate, ammonium chloride, ammonium phosphate, ammonium carbonate, and ammonium nitrate, but these are not limited thereto. These nitrogen sources may also be used alone or in a mixture.

Phosphorus sources that may be used in the present invention may include potassium dihydrogen phosphate or dipotassium hydrogen phosphate, or corresponding sodium-containing salts, but these are not limited thereto. In addition, the culture medium may contain a metal salt such as magnesium sulfate or iron sulfate, which is required for the growth. Lastly, in addition to the above-described substances, essential growth substances such as amino acids and vitamins may be used. Additionally, suitable precursors may be used in the culture medium. These substances may be appropriately added to the medium during culturing in a batch or continuous manner.

Basic compounds such as sodium hydroxide, potassium hydroxide, or ammonia, or acidic compounds such as phosphoric acid or sulfuric acid may be added to the culture medium in a suitable manner to adjust the pH of the culture medium. In addition, an anti-foaming agent such as fatty acid polyglycol ester may be used to suppress the formation of bubbles. In order to maintain the culture medium in an aerobic state, oxygen or oxygen-containing gas may be injected into the culture medium. The temperature of the culture medium may be usually 20°C to 45°C, preferably 25°C to 40°C, but may be changed depending on conditions and is not limited thereto.

In one specific embodiment of the present invention, the recombinant expression vectors (i.e., pCB-SP7.2-Pem, pCB-Clus-Pem, pCB-Pig-Pem, and pCB-CC-Pem) were introduced into the host CHO cells (ExpiCHO-S™ cells) and cultured in 30 mL of an ExpiCHO expression medium (CHO expression medium) for 12 days via a fed-batch culture (Example 4).

The method of the present invention for producing a target protein may include a step of recovering the target protein from the culture medium. As used herein, the term "recovery" is a process of obtaining the target protein from the culture medium, and may be performed using methods known in the art, for example, centrifugation, filtration, anion-exchange chromatography, crystallization, HPLC, *etc.,* but the method is not limited thereto.

The recovery step may include a purification process, and those skilled in the art may select and utilize among various known purification processes as needed. For example, the host cell can be separated from the culture medium or culture supernatant of the host cell by the conventional chromatographic methods such as immunoaffinity chromatography, receptor affinity chromatography, hydrophobic interaction chromatography, lectin affinity chromatography, size-exclusion chromatography, cation or anion exchange chromatography, high performance liquid chromatography (HPLC) and reversed-phase HPLC. In addition, when the desired protein is a fusion protein with a specific tag, label, or chelate moiety, it can be purified by a specific binding partner or drug. The purified protein may be cleaved into a desired protein region, such as the removal of the secretory factor, or it can remain as it is. A desired form of protein including additional amino acid may be produced by cutting the fusion protein during the cutting process.

The protein secretory factor consisting of the amino acid sequence of SEQ ID NO: 1, SEQ ID NO: 2, SEQ ID NO: 3, SEQ ID NO: 4, or SEQ ID NO: 5 of the present invention may be a secretory factor that is accurately cleaved at the N-terminal cleavage site of the target protein.

The signal peptide is located at the N-terminal region of the recombinant protein to be produced, and when the target protein is translocated, it is degraded by a signal peptidase. However, existing protein secretory factors can often degrade the quality of target proteins due to the mis-cleavage problem. The "mis-cleavage" refers to a phenomenon where the signal peptide is not completely degraded at the correct position and the signal peptide sequence partially remains at the N-terminus of the target protein.

In one specific embodiment of the present invention, the cleavage of the protein secretory factors (signal peptides) was confirmed using the purified target proteins by a Q-TOF MS mass spectrometer. As a result, it was confirmed that 100% cleavage was observed at the predicted cleavage sites.

Accordingly, the expression vector including the protein secretory factor (signal peptide) of the present invention increases the productivity of the target protein through efficient expression and secretion of the recombinant protein, and can be a powerful genetic tool to solve the mis-cleavage problem.

## [Advantageous Effects]

The protein secretory factor of the present invention, that is, the signal peptide can significantly increase the productivity of recombinant proteins through high-level expression and can be expected to be used as a powerful genetic tool, which can solve the mis-cleavage problem of the conventional signal peptides by 100% cleavage at cleavage sites.

## [Brief Description of Drawings]

Fig. 1 is a graph predicting the signal peptides using SignalP4.1.
Fig. 2 is a diagram confirming the expression levels of signal peptide-mCherry through temporary expression.
Fig. 3 is a diagram showing a vector map for site specific integration.
Fig. 4 is a diagram comparing the expression levels of mCherry in the site-specific integrated cells.
Fig. 5 is a graph showing mass data that confirms the cleavage of an anti-PD-1 antibody fused with SP7.2 and Clus.

## [Detailed Description of Preferred Embodiments]

Hereinafter, the present invention will be described in more detail by way of Examples. However, these Examples are given for illustrative purposes only, and the scope of the invention is not intended to be limited to or by these Examples

### Example 1. Preparation of Novel Signal Peptide Sequences Derived from CHO Cells

### 1-1. CHO HCP Mass Analysis

Four types (ADH, BSA, PHO, and ENL) of MassPREPTM Protein Digest Standard were added to the culture medium of CHO cells (DXB11) treated with trypsin as follows. Among the four types of MassPREPTM Protein Digest Standard, PHO was used as an internal standard for calculating the concentration of each host cell protein (HCP). Each sample was analyzed for host cell protein (HCP) using a 2D LC (high pH RP/Low pH RP)-Q-TOF (UDMSe) method.

MS data (UDMSe) was obtained for each fraction by injecting directly into Q-TOF MS in the 2D column. The MS data (UDMSe) of the 10 fractions obtained in the above manner were merged into one data using ProteinLynx Global SERVER (PLGS, Ver. 3.0.2) Software. Thereafter, the HCP was identified using the merged data of each sample and the Chinese Hamster Protein Database, and the concentration of each HCP was calculated using PHO as an internal standard.

### 1-2. Selection of Signal Peptides from CHO HCP Data

The proteins were arranged in the order of high concentrations, and the amino acid sequence of each protein was confirmed from the CHO Genome Database (http://chogenome.org). The thus-obtained amino acid sequences were entered into SignalP4.1 Server (http://www.cbs.dtu.dk/services/SignalP/) to predict the presence of secretory proteins and signal peptide sequences (Table 1, Figs. 1 and Table 4).

[Table 1]

| HCP Secretion Protein Predicted via SignalP4.1 | | | | |
|---|---|---|---|---|
| **Protein** | **SEQ ID NO:** | **SP (Signal Peptide) Sequence** | **SEQ ID NO:** | **DNA Sequence** |
| **Cathepsin B (Cat)** | 1 | MWWSLIPLSC LLALASA | 11 | ATG TGG TGG TCC TTG ATT CCG CTC TCT TGC CTG CTG GCA CTG GCA AGT GCC |
| **C-C motif chemokine** | 2 | MQFSARTLLC LLLTVAACSI YVLA | 12 | ATG CAG TTC TCC GCA AGA ACG CTT CTG TGC CTG CTA CTC ACA GTT GCT GCC TGT AGC ATC TAT GTG CTG GCC |
| **Nucleobindin-2 (Nuc)** | 3 | MRWKIIQLQY CFLLVPCMLT ALEA | 13 | ATG AGG TGG AAG ATC ATC CAG CTA CAG TAC TGT TTT CTT TTG GTC CCG TGC ATG CTT ACT GCT CTG GAA GCT |
| **Clusterin (Clus)** | 4 | MKILLLCVGL LLTWDNGMVL G | 14 | ATG AAG ATT CTC CTG TTG TGC GTG GGG CTG CTG CTG ACC TGG GAC AAT GGC ATG GTC CTG GGA |
| **Pigment epithelium-der ived factor (Pig)** | 5 | MQALVLLLWT GALLGHGSS | 15 | ATG CAG GCC CTG GTG CTA CTC CTC TGG ACA GGA GCC CTG CTT GGG CAT GGC AGC AGC |

(continued)

| HCP Secretion Protein Predicted via SignalP4.1 | | | | |
|---|---|---|---|---|
| **Protein** | **SEQ ID NO:** | **SP (Signal Peptide) Sequence** | **SEQ ID NO:** | **DNA Sequence** |
| **Procollagen C-endopeptida se enhancer 1(Proco)** | 6 | MLPAVLTSLL GPFLVAWVLP LARG | 16 | ATG CTG CCT GCT GTC CTA ACC TCC CTC CTG GGG CCA TTC CTT GTG GCC TGG GTA CTG CCT CTT GCC CGA GGC |
| **Sulfhydryl oxidase (Sulf)** | 7 | MRRCGRHSGS PSQMLLLLLP PLLLAVPGAG A | 17 | ATG AGG AGG TGC GGC CGC CAC TCG GGG TCG CCG TCG CAG ATG CTA CTG CTG CTG CTG CCG CCG CTG CTG CTC GCG GTG CCC GGC GCT GGC GCG |
| **Lipoprotein lipase (Lip)** | 8 | MESKALLLVA LGVWLQSLTA | 18 | ATG GAG AGC AAA GCC CTG CTC CTG GTG GCT CTG GGA GTG TGG CTC CAG AGT TTG ACC GCC |
| **Nidogen-1 (Nid)** | 9 | MLDASGWKPA AWTWVLLLQL LLAGPGDCLS | 19 | ATG CTG GAC GCG AGC GGC TGG AAG CCC GCG GCG TGG ACA TGG GTG CTG CTG CTG CAG CTA TTG CTG GCG GGG CCC GGA GAC TGC CTG AGC |

(continued)

| HCP Secretion Protein Predicted via SignalP4.1 | | | | |
|---|---|---|---|---|
| **Protein** | **SEQ ID NO:** | **SP (Signal Peptide) Sequence** | **SEQ ID NO:** | **DNA Sequence** |
| **Protein disulfide-isom erase (Pro)** | 10 | MDDRLLTVLL LLLGVSGPWG QG | 20 | ATG GAT GAT CGG CTC CTG ACA GTG TTG CTG CTC CTG CTG GGT GTC TCA GGC CCA TGG GGA CAG GGA |

### Example 2. Selection of CHO-Derived High Efficiency Signal Peptides via Temporary Expression

#### 2-1. Preparation of Recombinant Protein Expression Vectors for Temporary Expression

In order to confirm whether the 10 types of signal peptides selected in Example 1 can be used as general secretory factors, mCherry (pmCherry Vector, Clontech, 632522) protein was selected as the target protein.
The sequence of the polynucleotide of the gene encoding the mCherry protein is shown in Table 2.

[Table 2]

| Protein | SEQ ID NO: | Amino Acid Sequence | SEQ ID NO: | DNA Sequence |
|---|---|---|---|---|
| mCherry | 31 | MVSKGEEDN<br>MAIIKEFMRFK<br>VHMEGSVNG<br>HEFEIEGEGE<br>GRPYEGTQTA<br>KLKVTKGGPL<br>PFAWDILSPQ<br>FMYGSKAYVK<br>HPADIPDYLKL<br>SFPEGFKWE<br>RVMNFEDGG<br>VVTVTQDSSL<br>QDGEFIYKVK<br>LRGTNFPSDG<br>PVMQKKTMG | 32 | ATGGTGAGCAAGGGCGAGGAGGATAAC<br>ATGGCCATCATCAAGGAGTTCATGCGCT<br>TCAAGGTGCACATGGAGGGCTCCGTGA<br>ACGGCCACGAGTTCGAGATCGAGGGCG<br>AGGGCGAGGGCCGCCCCTACGAGGGC<br>ACCCAGACCGCCAAGCTGAAGGTGACC<br>AAGGGTGGCCCCCTGCCCTTCGCCTGG<br>GACATCCTGTCCCCTCAGTTCATGTACG<br>GCTCCAAGGCCTACGTGAAGCACCCCG<br>CCGACATCCCCGACTACTTGAAGCTGTC<br>CTTCCCCGAGGGCTTCAAGTGGGAGCG<br>CGTGATGAACTTCGAGGACGGCGGCGT<br>GGTGACCGTGACCCAGGACTCCTCCCT<br>GCAGGACGGCGAGTTCATCTACAAGGT<br>GAAGCTGCGCGGCACCAACTTCCCCTC |

(continued)

| Protein | SEQ ID NO: | Amino Acid Sequence | SEQ ID NO: | DNA Sequence |
|---|---|---|---|---|
| | | WEASSERMY<br>PEDGALKGEI<br>KQRLKLKDGG<br>HYDAEVKTTY<br>KAKKPVQLPG<br>AYNVNIKLDIT<br>SHNEDYTIVE<br>QYERAEGRH<br>STGGMDELYK | | CGACGGCCCCGTAATGCAGAAGAAGAC<br>CATGGGCTGGGAGGCCTCCTCCGAGCG<br>GATGTACCCCGAGGACGGCGCCCTGAA<br>GGGCGAGATCAAGCAGAGGCTGAAGCT<br>GAAGGACGGCGGCCACTACGACGCTGA<br>GGTCAAGACCACCTACAAGGCCAAGAA<br>GCCCGTGCAGCTGCCCGGCGCCTACAA<br>CGTCAACATCAAGTTGGACATCACCTCC<br>CACAACGAGGACTACACCATCGTGGAA<br>CAGTACGAACGCGCCGAGGGCCGCCA<br>CTCCACCGGCGGCATGGACGAGCTGTA<br>CAAGTAA |

Based on the gene encoding the mCherry protein, PCR was performed using the signal peptide sequences identified from the CHO HCP Mass Data and primers containing KpnI/XhoI, and the mCherry expressed by the 10 types of signal peptide sequences was constructed.

When the length of the signal peptide was long, the primer was divided into two and PCR was performed twice. The mCherry PCR products containing the 10 types of signal peptide sequences were cleaved with KpnI and XhoI and then cloned into pcDNA3.1(+) (Invitrogen, Cat. No. V790-20) to construct expression vectors.

Additionally, for use as a positive control, a mCherry protein expression vector fused with the SP7.2 signal peptide (Korean Patent Laid-Open Publication 10-2015-0125402 A), which is a known secretory factor, was prepared in the same manner. The sequence of the SP7.2 signal peptide is shown in Table 3.

[Table 3]

| Protein | SEQ ID NO: | SP Sequence | SEQ ID NO: | DNA Sequence |
|---|---|---|---|---|
| **SP7.2** | 33 | MHRPEAMLLLLTLALLGGPTWA | 34 | ATGCACCGGCCAGAGGCCATGCTGCTGCTGCTCACGCTTGCCCTCCTGGGGGCCCCACCTGGGCA |

[Table 4]

| Protein | GeneBank No. | Amino Acid Seqence | Expected SP Sequence from SignalP4.1 | DNA Sequence |
|---|---|---|---|---|
| Clusterin | XP_007643887.1 | 1 MKILLLCVGL LLTWDNGMVL GEQEVSDNEL KEMSTQGSRY INKEIQNAVQ GVKQIKTLIE<br>61 KTNEERKSLL NSLEEAKKKK EDALDDTRDT EMKLKAFPEV CNETMMALWE ECKPCLKQTC<br>121 MKFYARVCRS GSGLVGRQLE EFLNQSSPFY FWMNGDRIDS LMESDRQQSQ VLDAMQDSFT<br>181 RASGIMDMLF QDRFFTHEPQ DTHYFSPFGF PHRRPHFLYP KSRLVRSLIP LSHYGPPSFH<br>241 DMFQPFLEMI HQAQQAMDVQ FHRPAFQFPD KGLREGEDDR AVCKEIRHNS TGCLKMKGQC<br>301 EKCQEILSVD CSANNPAQAH LRQELNDSLQ MAERLTQQYN ELLHSLQTKM LNTSSLLEQL<br>361 NEQFNWVSQL ANLTQGEDQY YLRVSTVTTH SNNSEEPSRV TEVVVKLFDS DPITVVLPEE<br>421 VSKDNPKFMD TVAEKALQEY RKKSRAE | MKILLLCVGLLLTWDNGMVLG | ATG AAG ATT CTC CTG TTG TGC GTG GGG CTG CTG CTG ACC TGG GAC AAT GGC ATG GTC CTG GGA |
| Sulfhydryl oxidase | XP_007639037.1 | 1 MRRCGRHSGS PSQMLLLLLP PLLLAVPGAG AVQVSVLYSS SDPVTVLNAN TVRSTVLRSN<br>61 GAWAVEFFAS WCGHCIAFAP TWKELAYDVR EWRPVLNLAV LDCAEETNTA VCRDFNISGF<br>121 PTVRFFKAFS KNGSGITLPV ADASVETLRR KLIDALESHS DMWSSSRPKL KPAKLVEINE<br>181 FFAETNEDYL VLIFEDKDSY VGREVTLDLF QHHIPVHRVL NTERNAVSKF GVVEFPSCYL<br>241 LFRNGSFSRV PVVMESRLFY TSYLKGMSGP ILVDPPTTTI STDAPVTTDV VPTVWKVANH<br>301 ARIYMADLES SLHYIFLVEV GKFSVLEGQR LLALKKLVAV LAKYFPGRPL AQNFLHSIHD<br>361 WLQRQQRKKI PYKFFRAALD NRKEGIVLTE KVNWVGCQGS KPHFRGFPCS LWILFHFLTV<br>421 QASRYSENHP QEPADGQEVL QAMRSYVQWF FGCRDCAEHF ENMAASTMHR VRSPTSAVLW<br>481 LWTSHNKVNA RLSGAPSEDP YFPKVQWPLR ELCFDCHNEI NGREPVWDLE ATYRFLKAHF<br>541 SSENIILDTP VAGLATQRNP QILGATPEPH M | MRRCGRHSGSPSQMLLLLLPPLLLAVPGAGA | ATG AGG AGG TGC GGC CGC CAC TCG GGG TCG CCG TCG CAG ATG CTA CTG CTG CTG CTG CCG CCG CTG CTG CTC GCG GTG CCC GGC GCT GGC GCG |

| | | | | |
|---|---|---|---|---|
| Cathepsin B | XP_003498026.1 | 1 MWWSLIPLSC LLALASAHNK PSFHPLSDDL INYINKRNTT WQAGRNFHNV DISYLKRLCG<br>61 TIMGGPKLPE RVAFAEDMEL PENFDAREQW SNCPTIKQIR DQGSCGSCWA FGAVGAMSDR<br>121 LCIHTNGHVN VEVSAEDLLT CCGSQCGDGC NGGYPSGAWN FWIKKGLVSG GLYNSHVGCL<br>181 PYTIPPCEHH VNGSRPQCTG EGDTPKCTKS CEAGYSPSYK EDKHYGYTSY SVSNNEKEIM<br>241 AEIYKNGPVE GAFTVFSDFL TYKSGVYKHE AGDIMGGHAI RILGWGVENS VPYWLVANSW<br>301 NVDWGDNGLF KILRGEDHCG IESEIVAGIP RTDLYWGRF | MWWSLIPLSC LLALASA | ATG TGG TGG TCC TTG ATT CCG CTC TCT TGC CTG CTG GCA CTG GCA AGT GCC |
| Nucleobindin-2 | XP_003513452.1 | 1 MRWKIIQLQY CFLLVPCMLT ALEAVPIDVD KTKVHNTEPV ESARIDPPDT GLYYDEYLKQ<br>61 VIDVLETDQH FREKLQKADI EEIRSGRLSK ELDLVSHHVR TKLDELKRQE VARLRMLIKA<br>121 KLDSLQDTGM NHHLLLKQFE HLNHQNPDTF ESSDLDMLIK AATADLEQYD RTRHEEFKKY<br>181 EMMKEHERRE YLKTLNEEKR KEEESKFEEM KRKHENHPKV NHPGSKDQLK EVWEEADGLD<br>241 PNDFDPKTFF KLHDVNNDGF LDEQELEALF TRELEKVYDP RNAEDDMIEM EEERLRMREH<br>301 VMNEIDNNKD RLVTLEEFLR ATEKKEFLEP DSWETLGQQQ LFTEEELKEY ESIIAMQENE<br>361 LKKRADELQK QKEELQRQHD HLEAQKQEYQ QAVQQLEHKK FQQGIAPSGP AGELEFKPRM | MRWKIIQLQY CFLLVPCMLT ALEA | ATG AGG TGG AAG ATC ATC CAG CTA CAG TAC TGT TTT CTT TTG GTC CCG TGC ATG CTT ACT GCT CTG GAA GCT |
| Procollagen C-endopeptidase enhancer 1 | XP_003510679.1 | 1 MLPAVLTSLL GPFLVAWVLP LARGQTPNYT RPVFLCGGDV TGESGYVASE GFPNLYPPNK<br>61 KCIWTITVPE GQTVSLSFRV FDMELHPSCR YDALEVFAGS GTSGQRLGRF CGTFRPAPVV<br>121 APGNQVTLRM TTDEGTGGRG FLLWYSGRAT SGTEHQFCGG RMEKAQGTLT TPNWPESDYP<br>181 PGISCSWHII APSDQVIMLT FGKFDVEPDT YCRYDSVSVF NGAVSDDSKR LGKFCGDKAP<br>241 SPISSEGNEL LVQFVSDLSV TADGFSASYK TLPRDAVEKE LAPSPGEDVQ LGPQSRSDPK<br>301 TGTGPKVKPP SKPKFQPAEK PEVSPDTQET PVAPDPPSAT CPKQYKRLGT LQSNFCASSL<br>361 VVTGTVKTMV RGPGEGLTVT ISLLGVYKSG GLDLPSPPTD TSLKLYVPCR QMPPMKKGAS<br>421 YLLMGQVEEN RGPILPPESF LVPYKPNQDQ ILNNLRKRKC PSQPRPAA | MLPAVLTSLL GPFLVAWVLP LARG | ATG CTG CCT GCT GTC CTA ACC TCC CTC CTG GGG CCA TTC CTT GTG GCC TGG GTA CTG CCT CTT GCC CGA GGC |

| | | | | |
|---|---|---|---|---|
| **-C motif chemokine** | XP_003495840.1 | 1 MQFSARTLLC LLLTVAACSI YVLAQPDAVN SPLTCCYSFT AKRIPEKRLE SYKRITSSKC<br>61 PKEAVIFITK LKREICADPK QDWVQTYTKK LDQSQAKSEA ATVYKTAPLN ANLTHESAVN<br>121 ASTTAFPTTD LRTSVRVTSM TVN | MQF SARTLLC LLLTVAACSI YVLA | ATG CAG TTC TCC GCA AGA ACG CTT CTG TGC CTG CTA CTC ACA GTT GCT GCC TGT AGC ATC TAT GTG CTG GCC |
| **ipoprotein lipase** | XP_003499976.1 | 1 MESKALLLVA LGVWLQSLTA SQGXAAADGG RDFTDIESKF ALRTPDDTAE DNCHLIPGIA<br>61 ESVSNCHFNH SSKTFVVIHG WTVTGMYESW VPKLVAALYK REPDSNVIVV DWLYRAQQHY<br>121 PVSAGYTKLV GNDVARFINW MEEEFNYPLD NVHLLGYSLG AHAAGVAGSL TNKKVNRITG<br>181 LDPAGPNFEY AEAPSRLSPD DADFVDVLHT FTRGSPGRSI GIQKPVGHVD IYPNGGTFQP<br>241 GCNIGEAIRV IAERGLGDVD QLVKCSHERS IHLFIDSLLN EENPSKAYRC NSKEAFEKGL<br>301 CLSCRKNRCN NVGYEINKVR AKRSSKMYLK TRSQMPYKVF HYQVKIHFSG TESDKQLNQA<br>361 FEISLYGTVA ESENIPFTLP EVSTNKTYSF LIYTEVDIGE LLMMKLKWKS DSYFSWSDWW<br>421 SSPGFVIEKI RVKAGETQKK VIFCAREKVS HLQKGKDSAV FVKCHDKSLK KSG | MES KALLLVA LGVWLQSLTA | ATG GAG AGC AAA GCC CTG CTC CTG GTG GCT CTG GGA GTG TGG CTC CAG AGT TTG ACC GCC |
| **idogen-1** | XP_003507635.2 | 1 MLDASGWKPA AWTWVLLLQL LLAGPGDCLS RQELFPFGPG QGDLELEAGD DVVSPALELI<br>61 GELSFYDRSD ITSVYVTTNG IIAMSEPPAR ESHPGTFPPS FGSVAPFLAD LDTTDGLGNV<br>121 YYREDLSPSI MQMAAEYVQR GFPEVPFQPT SVVVVTWESV APYEGPSGSS AQEGKRNTFQ<br>181 AVLASSNSSS YAIFLYPEDG LQFFTTFSKK DENQVPAMVG FSQGLVGFLW RSDGAYNIFA<br>241 NDRESIENLA KSSNAGHQGV WVFEIGSPAT AKGVVSADVN LGLDDDGSDY EDEEYDLATS<br>301 HLGLEDMATQ PFPSPSPRRG NTHPHDVPRV LSPSYEATER PHGIPTERTR TFQLPAERFH<br>361 QQHPQVIDVD EVEETGIVFS YNIGSQQTCA NNRHQCSVHA ECRDYATGFC CRCVANYTGN<br>421 GRQCVAEGSP QRVNGKVKGR IFVGNSQVPV VFENTDLHSY VVMNHGRSYT AISTIPETVG<br>481 YSLLPLAPIG GIIGWMFAVE QNGFKNGFSI TGGEFTRQAE VTFLGHPGKL VLKQHFSGID<br>541 EHGHLTINTE LDGRVPQIPY GSSVHIEPYT ELYHYSSSVI TSSSTREYTV TEPDPDGTAP<br>601 SHTHVYQWRQ TITFQECVHD DSRPALPSTQ QLSVDSVFVL YNQEERILRY ALSNSIGPVR<br>661 EGSPDALQNP CYIGTHGCDS NAACRPGPGT QFTCECSIGF | MLD ASGWKPA AWTWVLLLQL LLAGPGDCLS | ATG CTG GAC GCG AGC GGC TGG AAG CCC GCG GCG TGG ACA TGG GTG CTG CTG CTG CAG CTA TTG CTG GCG GGG CCC GGA GAC TGC CTG AGC |

| | | | | |
|---|---|---|---|---|
| | | RGDGQTCYDI DECSEQPSRC<br>721 GNHAACNNSP GAYLCECVEG YHFSDGGICV ADVDQRPINY CETGLHNCDI PQRAQCIYMG<br>781 GSSYTCSCLP GFSGDGRACQ DVDECQLSRC HPDAFCYNTP GSFTCQCKPG YQGDGFQCVP<br>841 GEVGKTRCQL EREHILGASG VADAQQPRLL GMYVPQCDEY GHYEPTQCHH GTGYCWCVDR<br>901 DGRELEGTRT QPGMRPPCLS TVAPPIHQRP VVPTAVIPLP PGTHLLFAQT GKIERLPLEG<br>961 NTMKKTEAKA FFHIPAKVII GLAFDCVDKV VYWTDISEPS IGRASLHGGE PTTIIRQDLG<br>1021 SPEGIALDHL GRNIFWTDSQ LDRIEVARMD GTQRRVLFDT GLVNPRGIVT DSVGGNLYWT<br>1081 DWNRENPKIE TSYMDGTNRR ILAQDNLGLP NGLTFDAFSS QLCWVDAGTH RAECLNPAQP<br>1141 SSRKVLEGLQ YPFAVTSYGK NLYYTDWKTN SVIAMDLAIS KEMDAFTPTS RPGYMASPLP<br>1201 CPNALKATTT AQ | | |
| Pigment epithelium-derived factor | XP_003515170.1 | 1 MQALVLLLWT GALLGHGSSQ NVASSSEEGS PAPDSTGEPV EEEEDPFFKV PVNKLAAAVS<br>61 NFGYDLYRLR SSASPTANVL LSPLSVATAL SALSLGAEQR TESIIHRALY YDLISNSDIH<br>121 STYKELLASV TAPEKSLKSA SRIVFERKLR VRSSFVAPLE KSYGTRPRIL TGNPRIDLQE<br>181 INNWIQAQMK GKLARSTREM PSAISILLLG VAYFKGQWVT KFDSRKTSLQ DFHLDEDRTV<br>241 KVPMMSEPKA ILRYGLDSDL NCKVWEHGGW EGSERGRVSS IRKSIWGYSK IHELQSLFES<br>301 PDFSKITGKP VKLTQVEHRA AFEWNEEGVE TSPNPGLQPV RLTFPLDYHL NQPFIFVLRD<br>361 TDTGALLFIG KILDPRGT | MQALVLLLWT GALLGHGSS | ATG CAG GCC CTG GTG CTA CTC CTC TGG ACA GGA GCC CTG CTT GGG CAT GGC AGC AGC |
| Protein disulfide-isomerase | XP_003501525.1 | 1 MDDRLLTVLL LLLGVSGPWG QGQEPEGPSE VLPEESSGEE VPKEDGILVL SHHTLSLALQ<br>61 EHPALMVEFY APWCGHCKAL APEYSKAAAL LAAESASVTL AKVDGPAEPE LTKEFGVVGY<br>121 PTLKFFQNGN RTNPEEYTGP QKAEGIAEWL RRRVGPSAKR LEDEEDVQAL TDKWEVVVIG<br>181 FFQDLQGEDV ATFLALARDA LDITFGFTDQ PQLFQKFGLT KDTVILFKKF DEGRADFPVD<br>241 KDTGLDLGDL SRFLVTHSMH LVTEFNSQTS PKIFAAKILN HLLLFVNKTL AQHRELLTDF<br>301 REAAPPFRGQ VLFVMVDVAA DNDHVLNYFG LKAEEAPTLR LINVETTKKY APTGLVPITA<br>361 ASVAAFCQAV LHGQVKPYLL SQEIPPDWDE RPVKTLVGKN FEQVAFDETK NVFVKFYAPW<br>421 CSHCKEMAPA WEALAEKYRD REDIVIAELD ATANELEAFS VHGYPTLKFF PAGPDRKVIE<br>481 YKSTRDLETF SKFLDSGGNL PEEEPKEPAI STPEIQDNST VGPKEEL | MDDRLLTVLL LLLGVSGPWG QG | ATG GAT GAT CGG CTC CTG ACA GTG TTG CTG CTC CTG CTG GGT GTC TCA GGC CCA TGG GGA CAG GGA |

### 2-2. Temporary Expression

Each of the mCherry expression vectors expressed by the 10 types of signal peptides were transfected (1 mL) according to the CHO-S cell line Amaxa 4D-Nucleofector protocol. Thereafter, on the 2nd and 6th days, the fluorescence

values of the intracellular fluorescence and the fluorescent proteins secreted from the culture medium were measured (Fig. 3).

In the case of the intracellular fluorescence, FACS (Accuri) was used to measure the average value in the histogram of the portion higher than that of the negative control (empty vector, pMaxGFP).

In the case of the fluorescence values of the fluorescent proteins secreted into the culture medium, 100 μ0 was sampled on the 2nd and 6th days, and then centrifuged to obtain the supernatant only, and fluorescence was measured at 587/610 nm using a multiple reader.

It was confirmed that the signal peptides with a high fluorescence value measured in the culture medium were Cat, CC, Nuc, Clus, and Pig. Additionally, the four types of secretory factors (Clus, Pig, Nuc, and CC) which showed the expression higher than the positive control SP7.2, SP7.2 to be used as the positive control, and one type of signal peptide with a high fluorescence value in cells (Proco) were selected as a negative control.

**Example 3. Comparison of Expression through Site-Specific Integration**

**3-1. Construction of Expression Vectors for Site-Specific Integration**

The mCherry sequences including the 5 types of signal peptides (Clus, Pig, Nuc, CC, and Proco) selected in Example 2 and the control SP7.2 were identically inserted into a specific site of the CHO genome to quantitatively compare the expression levels (Figs. 4 and 5).

The insertion site was set at the Hprt Site, and a homology arm sequence and sgRNA sequence were designed with reference to J.S Lee et al., 2015, "Site-Specific integration in CHO cells mediated by CRISPR/Cas9 and homology-directed DNA repair pathway", Sci. Rep., 5.

In the case of the 5' homology arm, PCR was performed using primers containing Bg1 II and NruI enzyme sites along with the CHO-S genome as a template. Thereafter, it was cloned into a pcDNA3.1(+) vector digested with Bg1II/NruI.

In the case of 3' homology arm, PCR was conducted using each primer containing SalI site along with the CHO-S genome as a template, and then SalI single cut was made along with the vector inserted with 5' homology arm, and it was cloned into the downstream of the NeoR gene (pcDNA3.1_hprt).

In order to confirm only those that have been undergone homology recombination and inserted into the genome, Cmy-GFP-BHG pA fragments were constructed in the upstream region of the 5' homology arm and inserted into SpeI and Bg1 II (pcDNA3.1_G_hprt) for double selection.

In the case of the GFP fragment, it was first inserted into the MCS of the pcDNA3.1(+) Vector with NcoI/XbaI, and then PCR was performed using primers containing SpeI and Bg1II restriction sites. Thereafter, SpeI was inserted using the Bg1II site into the vector (pcDNA_hprt) containing the homology.

Using the completed pcDNA3.1_G_hprt Vector, the mCherry gene sequences containing the signal peptide sequence was cut with KpnI and XhoI and inserted into the MCS region.

As a result, the pcDNA3.1-based expression vector containing the expression cassette in the form of CMV-EGFP-pA-5' Hprt Homology Arm-CMV-signal peptide candidate-mCherry-BGH pA-NeoR selection marker cassette-3'Hprt homology arm was constructed.

**3-2. Site-Specific Integration**

Knock-in was performed using CRISPR-Cas9 in order to insert the 6 types of vectors for site specific integration into the Hprt Site in the CHO-S genome. 240 ng of sgRNA, 1,250 ng of cas9 protein, and 1 μg of donor vector were independently mixed with Nucleofector solution to prepare 50 μL mixture.

CHO-S $1\times10^6$ cells were first dissolved in 50 μL of Nucleofector and then mixed with the previously prepared mixture, and subsequently, the final 100 μL of the mixture was subjected to electroporation.

After performing the electroporation, the mixture was mixed with 0.5 mL of the medium, added to 2.5 mL of the medium, and cultured in a 6 well-plate at 36.5°C and 5% $CO_2$.

After 2 days, selection was performed in the CD CHO media containing Zeneticin (0.5 mg/L), and then the cells were sub-cultured until 90% of viability was recovered.

After 90% of viability was recovered, 4 mL of the cells were cultured in duplicate in a 6 well-plate at a concentration of $3\times10^5$ cells/mL, and the Vi-Cell and the medium fluorescence values (587 nm/610 nm) were measured every 2 to 3 days (Fig. 5).

As a result, it was confirmed that the CC, Clus, and Pig secretory peptides showed higher expression than that of the control SP7.2.

## Example 4. Expression of Anti PD-1 Antibody and Mass Analysis

### 4-1. Preparation of Expression Vectors for Production of Anti PD-1 Antibody

After comparing the expression ability of signal peptides through site specific integration, anti-PD-1 antibodies fused with the 4 types of signal peptides (CC, Pig, Clus, and SP7.2) including CC, Clus, and Pig showing high expression were expressed. The Pembrolizumab (Keytruda®) antibody sequence was used as the target protein.

DNA sequences corresponding to the amino acid sequences of the light chain and the heavy chain were synthesized, and subsequently, sequences fused with each of the signal peptide sequences were produced through overlap PCR.

In the case of the light chain, the amino acid sequences were restricted with BamHI and XhoI, and in the case of the heavy chain, the amino acid sequences were restricted with AscI and NotI, and then the antibodies were inserted into the pTz-D1G1 vector, a variant of pcDNA3.1(+) (including the promoter of KR Patent No. 10-1038126B1).

**pCB SP7.2 Pem**

'(N-terminal) - [BamHI Restriction Site - Signal Peptide (SEQ ID NO: 33) - Pem Light Chain (SEQ ID NO: 58) - XhoI Restriction Site] - (C-terminal)' / '(N-terminal) - [AscI Restriction Site - Signal Peptide (SEQ ID NO: 33) - Pem Heavy Chain (SEQ ID NO: 59) - NotI Restriction Site] - (C-terminal)'

**pCB Clus Pem**

'(N-terminal) - [BamHI Restriction Site - Signal Peptide (SEQ ID NO: 4) - Pem Light Chain (SEQ ID NO: 58) - XhoI Restriction Site] - (C-terminal)' / '(N-terminal) - [AscI Restriction Site - Signal Peptide (SEQ ID NO: 4) - Pem Heavy Chain (SEQ ID NO: 59) - NotI Restriction Site] - (C-terminal)'

**pCB CC Pem**

'(N-terminal) - [BamHI Restriction Site - Signal Peptide (SEQ ID NO: 2) - Pem Light Chain (SEQ ID NO: 58) - XhoI Restriction Site] - (C-terminal)' / '(N-terminal) - [AscI Restriction Site - Signal Peptide (SEQ ID NO: 2) - Pem Heavy Chain (SEQ ID NO: 59) - NotI Restriction Site] - (C-terminal)'

**pCB Pig Pem**

'(N-terminal) - [BamHI Restriction Site - Signal Peptide (SEQ ID NO: 5) - Pem Light Chain (SEQ ID NO: 58) - XhoI Restriction Site] - (C-terminal)' / '(N-terminal) - [AscI Restriction Site - Signal Peptide (SEQ ID NO: 5) - Pem Heavy Chain (SEQ ID NO: 59) - NotI Restriction Site] - (C-terminal)'

### 4-2. Expression of Anti-PD1 Antibody

The prepared recombinant expression vectors pCB-SP7.2-Pem, pCB-Clus-Pem, pCB-Pig-Pem and pCB-CC-Pem were introduced into ExpiCHO-S™ cells (Thermo Fisher Scientific), and cultured in the ExpiCHO expression medium (Thermo Fisher Scientific; 30 mL) for 12 days (Fed-Batch Culture; Day 1 & Day 5 Feeding) to express the fusion polypeptide (i.e., Pembrolizumab).

### 4-3. Purification of Anti-PD1 Antibody and Mass Analysis

The fusion polypeptide produced through the expression of the recombinant vectors was purified by ProteinA. Specifically, the recovered culture solution was filtered with a 0.22 μm filter, and then a column packed with ProteinA resin (Hitrap MSS, GE Healthcare, 11-0034-93) was mounted on AKTA™ Avant25 (GE Healthcare Life Sciences) and a PBS buffer was flowed through to equilibrate the column.

After the filtered culture solution was injected into a column, a PBS buffer was flowed through again to wash the column. After washing of the column was completed, an elution buffer (citrate buffer, pH 3.5) was flowed through the column to elute the target protein. The eluate was concentrated using the Amicon Ultra filter device (MWCO 30K, Merck) and a centrifuge. After the concentration was performed, buffer exchange was performed with PBS.

Quantitative analysis of the fusion polypeptide was performed by measuring the absorbance at 280 nm and 340 nm using a UV spectrophotometer (G113A, Agilent Technologies), and employing the following calculation equation. The extinction coefficient of each material was a value theoretically calculated using the amino acid sequence (1.404).

$$\text{Protein Concentration (mg / mL)} = \frac{\text{Absorbance } (A_{280\,nm} - A_{340\,nm})}{\text{*Extinction Coefficient}} \times \text{Dilution Factor}$$

(*Extinction Coefficient (0.1%): It is a theoretical absorbance at 280 nm under assumption that the protein concentration is 0.1% (1 g/L), and all cysteines on the primary sequence are oxidized to form a disulfide bond. Calculated via ProtParam tool (https://web.expasy.org/protparam/)

The purified target proteins were used to confirm the presence of mis-cleavage of signal peptides at the N-terminus of the proteins using Q-TOF MS (Figs 6). After dilution to a concentration of 1 mg/mL, the proteins were treated with PNGaseF, followed by 6M Guanidine and DTT, and then loaded onto Q-TOF MS (RMM-MT-001: ACQUITY UPLC+Q-TOF SYNAPT G2 (Waters)).

As a result, it was confirmed that 100% cleavage was observed at the predicted cleavage sites.

Based on the results, the signal peptide, which is the CHO cell-derived protein secretory factor of the present invention, improves productivity by increasing the expression level of the recombinant proteins, and by confirming through mass analysis that 100% cleavage was observed at the predicted cleavage sites, it implies that the signal peptide of the present invention can be a powerful genetic tool which can solve the mis-cleavage, which is the problem of the existing protein secretory factors.

While the present invention has been described with reference to the particular illustrative embodiments, it will be understood by those skilled in the art to which the present invention pertains that the present invention may be embodied in other specific forms without departing from the technical spirit or essential characteristics of the present invention. Therefore, the embodiments described above are considered to be illustrative in all respects and not restrictive. Furthermore, the scope of the present invention is defined by the appended claims rather than the detailed description, and it should be understood that all modifications or variations derived from the meanings and scope of the present invention and equivalents thereof are included in the scope of the appended claims.

## Claims

1. A protein secretory factor consisting of an amino acid sequence of SEQ ID NO: 1, SEQ ID NO: 2, or SEQ ID NO: 3.

2. The protein secretory factor of claim 1, wherein the protein is an endogenous protein or a foreign protein.

3. An expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked.

4. The expression cassette of claim 3, wherein the target protein is selected from the group consisting of antibody, antibody fragment (Fab or ScFv), fusion protein, protein scaffold, human growth hormone, serum protein, immunoglobulin, cytokine, $\alpha$-, $\beta$- or $\gamma$-interferon, granulocyte-macrophage colony-stimulating factor (GM-CSF), platelet-derived growth factor (PDGF), phospholipase-activating protein (PLAP), insulin, tumor necrosis factor (TNF), growth factor, hormone, calcitonin, calcitonin gene-related peptide (CGRP), enkephalin, somatomedin, erythropoietin, hypothalamic-releasing factor, growth differentiation factor, cell adhesion protein, prolactin, chorionic gonadotropin, tissue plasminogen activator, growth hormone-releasing peptide (GHPR), thymic humoral factor (THF), asparaginase, arginase, arginine deaminase, adenosine deaminase, peroxide dismutase, endotoxinase, catalase, chymotrypsin, lipase, uricase, adenosine diphosphatase, tyrosinase, bilirubin oxidase, glucose oxidase, glucodase, galactosidase, glucocerebrosidase, and glucuronidase.

5. The expression cassette of claim 3, wherein the nucleic acid sequence encoding the protein secretory factor consisting of the amino acid sequence of SEQ ID NO: 1, SEQ ID NO: 2, SEQ ID NO: 3, SEQ ID NO: 4, or SEQ ID NO: 5 consists of the nucleic acid sequence of SEQ ID NO: 11, SEQ ID NO: 12, SEQ ID NO: 13, SEQ ID NO: 14, or SEQ ID NO: 15.

**6.** The expression cassette of claim 3, wherein the expression cassette further comprises a nucleic acid sequence encoding any one of protein secretory factors consisting of an amino acid sequence of SEQ ID NO: 6, SEQ ID NO: 7, SEQ ID NO: 8, SEQ ID NO: 9, and SEQ ID NO: 10.

**7.** The expression cassette of claim 3, wherein the expression cassette expresses a target protein in which no additional amino acids have been added, from which the nucleic acid sequence encoding the secretory factor has been removed, when expressed in a cell, as it is.

**8.** An expression vector for secreting a target protein, comprising an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked.

**9.** The expression vector of claim 8, wherein the target protein is selected from the group consisting of antibody, antibody fragment (Fab or ScFv), fusion protein, protein scaffold, human growth hormone, serum protein, immunoglobulin, cytokine, α-, β- or γ-interferon, granulocyte-macrophage colony-stimulating factor (GM-CSF), platelet-derived growth factor (PDGF), phospholipase-activating protein (PLAP), insulin, tumor necrosis factor (TNF), growth factor, hormone, calcitonin, calcitonin gene-related peptide (CGRP), enkephalin, somatomedin, erythropoietin, hypothalamic-releasing factor, growth differentiation factor, cell adhesion protein, prolactin, chorionic gonadotropin, tissue plasminogen activator, growth hormone releasing peptide (GHPR), thymic humoral factor (THF), asparaginase, arginase, arginine deaminase, adenosine deaminase, peroxide dismutase, endotoxinase, catalase, chymotrypsin, lipase, uricase, adenosine diphosphatase, tyrosinase, bilirubin oxidase, glucose oxidase, glucodase, galactosidase, glucocerebrosidase, and glucuronidase.

**10.** The expression vector of claim 8, wherein the nucleic acid sequence encoding the protein secretory factor consisting of the amino acid sequence of SEQ ID NO: 1, SEQ ID NO: 2, SEQ ID NO: 3, SEQ ID NO: 4, or SEQ ID NO: 5 consists of the nucleic acid sequence of SEQ ID NO: 11, SEQ ID NO: 12, SEQ ID NO: 13, SEQ ID NO: 14, or SEQ ID NO: 15.

**11.** The expression vector of claim 8, wherein the expression vector further comprises a nucleic acid sequence encoding any one of protein secretory factors consisting of an amino acid sequence of SEQ ID NO: 6, SEQ ID NO: 7, SEQ ID NO: 8, SEQ ID NO: 9, and SEQ ID NO: 10.

**12.** The expression vector of claim 8, wherein the expression vector expresses a target protein in which no additional amino acids have been added, from which the nucleic acid sequence encoding the secretory factor has been removed, when expressed in a cell, as it is.

**13.** A transformed cell in which the expression vector of any one of claims 8 to 12 is introduced into a host cell.

**14.** The transformed cell of claim 13, wherein the host cell is a Chinese hamster ovary cell (CHO cell).

**15.** A method for producing a target protein, comprising:

i) culturing a transformed cell comprising an expression vector for secreting a target protein, which includes an expression cassette in which a nucleic acid sequence encoding a protein secretory factor consisting of an amino acid sequence of SEQ ID NO: 1, SEQ ID NO: 2, SEQ ID NO: 3, SEQ ID NO: 4, or SEQ ID NO: 5; and a gene encoding a target protein are operably linked; and
ii) recovering the target protein from the culture medium or culture supernatant of the cultured cells.

**16.** The method of claim 15, further comprising purifying the recovered target protein.

**17.** The method of claim 15, wherein the host cell is a Chinese hamster ovary cell (CHO cell).

**18.** The method of claim 15, wherein the protein secretory factor consisting of the amino acid sequence of SEQ ID NO: 1, SEQ ID NO: 2, SEQ ID NO: 3, SEQ ID NO: 4, or SEQ ID NO: 5 is cleaved at the N-terminal cleavage site of the target protein.

**19.** The method of claim 15, wherein the target protein is a target protein itself in which no additional amino acids have been added, from which the nucleic acid sequence encoding the secretory factor has been removed.

[Fig. 1a]

Clusterin

# SignalP-4.1 euk predictions

> Sequence

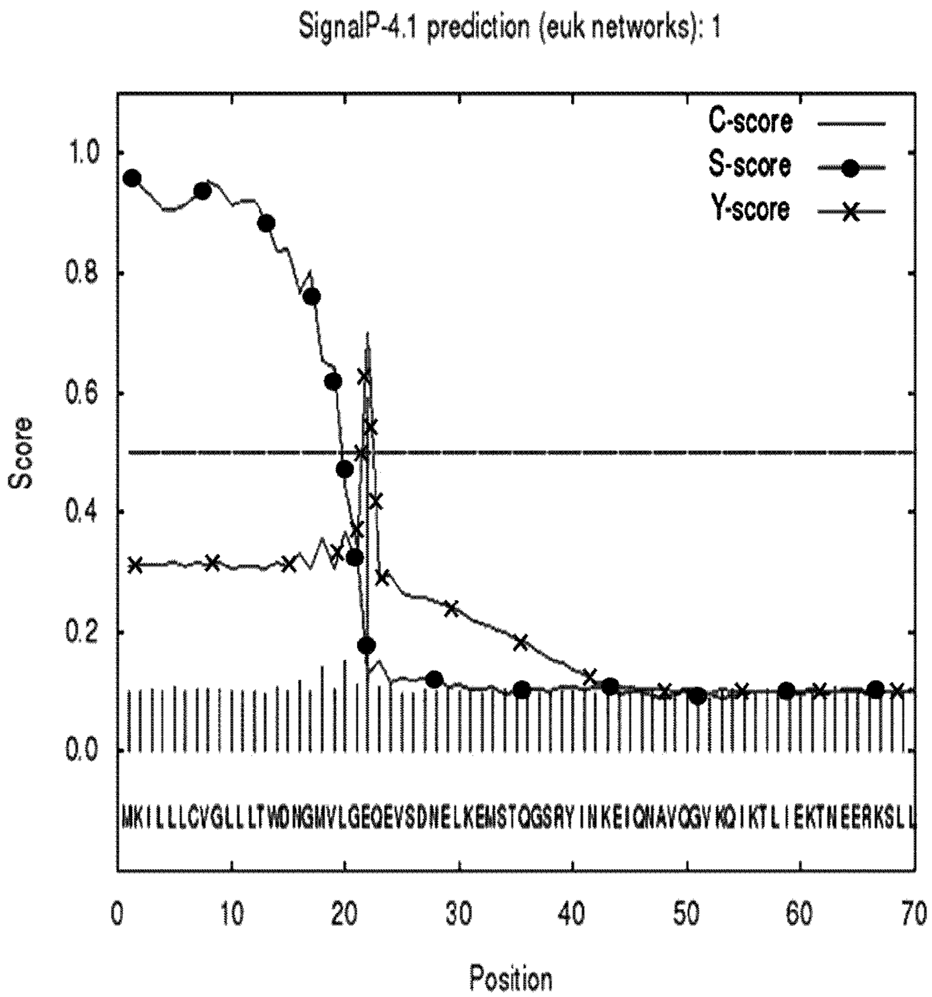


| # Measure | | Position | Value | Cutoff | signal peptide? |
|---|---|---|---|---|---|
| max. | C | 22 | 0.592 | | |
| max. | Y | 22 | 0.702 | | |
| max. | S | 1 | 0.962 | | |
| mean | S | 1-21 | 0.826 | | |
| | D | 1-21 | 0.769 | 0.450 | YES |

Name=Sequence SP='YES' Cleavage site between pos. 21 and 22: VLG-EQ D=0.769 D-cutoff=0.450 Networks=SignalP-noTM

[Fig. 1b]

Sulfhydryl oxidase

# SignalP-4.1 euk predictions

> Sequence

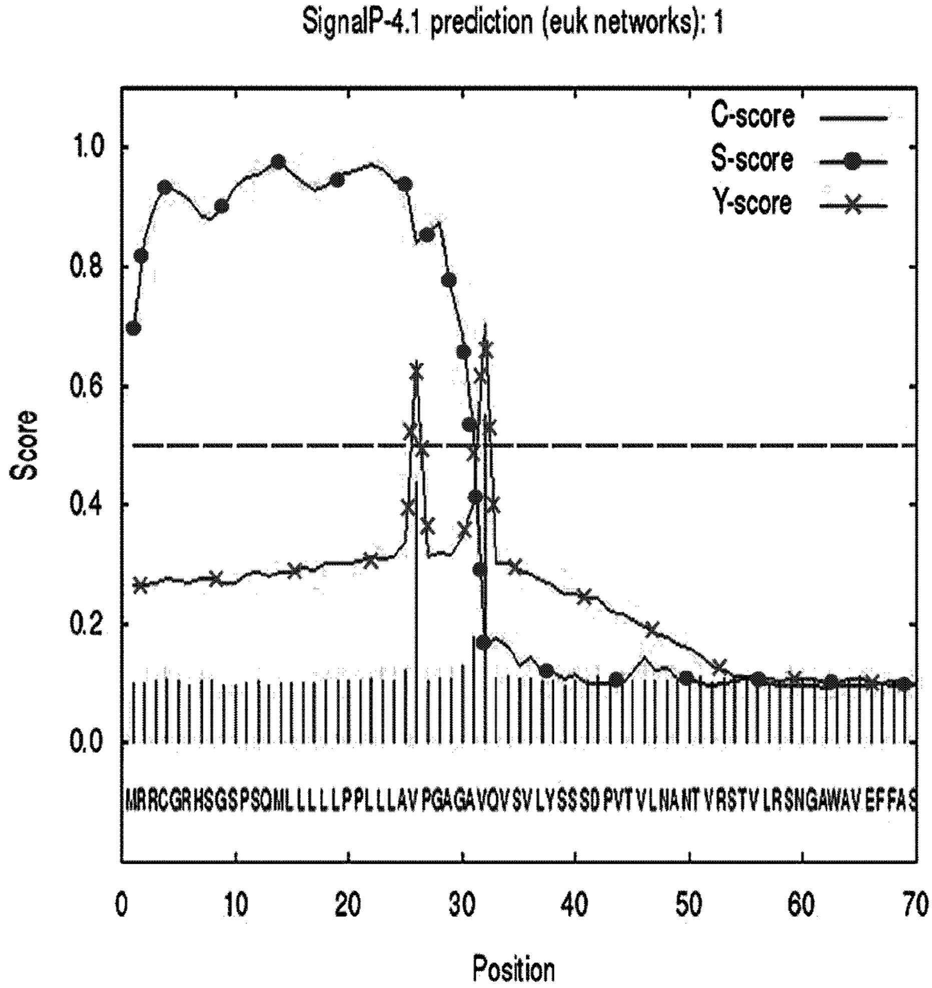


| # Measure | | Position | Value | Cutoff | signal peptide? |
|---|---|---|---|---|---|
| max. | C | 32 | 0.552 | | |
| max. | Y | 32 | 0.704 | | |
| max. | S | 14 | 0.974 | | |
| mean | S | 1-31 | 0.893 | | |
| | D | 1-31 | 0.806 | 0.450 | YES |

Name=Sequence SP='YES' Cleavage site between pos. 31 and 32: AGA-VQ D=0.806 D-cutoff=0.450 Networks=SignalP-noTM

[Fig. 1c]

## Cathepsin B

# SignalP-4.1 euk predictions
> Sequence

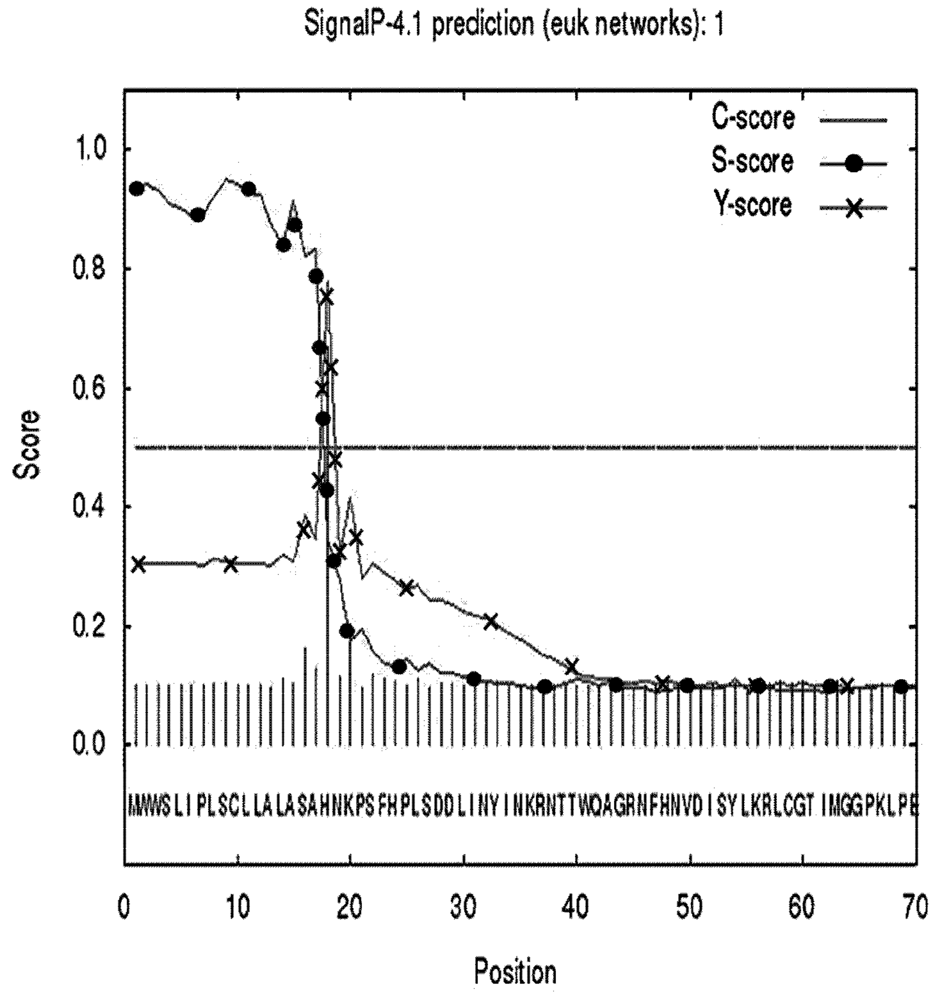


| # Measure | | Position | Value | Cutoff | signal peptide? |
|---|---|---|---|---|---|
| max. | C | 18 | 0.667 | | |
| max. | Y | 18 | 0.779 | | |
| max. | S | 9 | 0.951 | | |
| mean | S | 1-17 | 0.902 | | |
| | D | 1-17 | 0.846 | 0.450 | YES |

Name=Sequence SP='YES' Cleavage site between pos. 17 and 18: ASA-HN D=0.846 D-cutoff=0.450 Networks=SignalP-noTM

[Fig. 1d]

## Nucleobindin-2

# SignalP-4.1 euk predictions
> Sequence

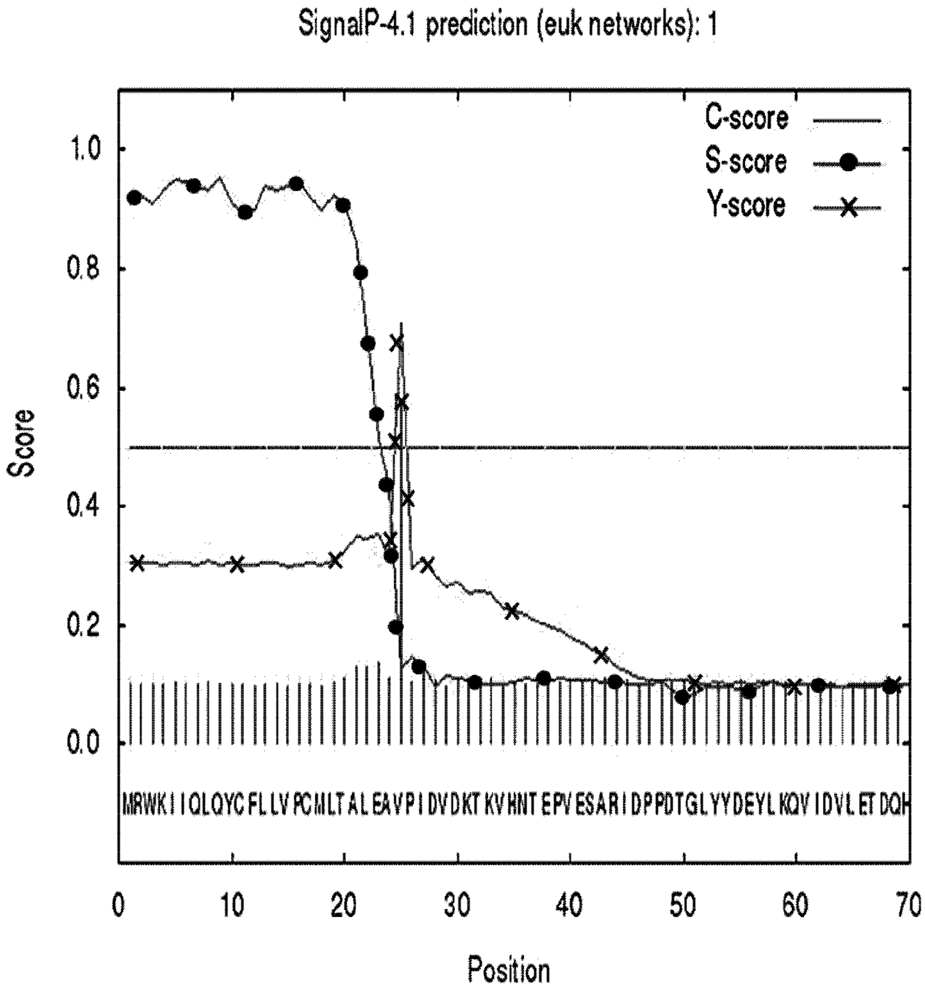


| # Measure | | Position | Value | Cutoff | signal peptide? |
|---|---|---|---|---|---|
| max. | C | 25 | 0.575 | | |
| max. | Y | 25 | 0.707 | | |
| max. | S | 9 | 0.954 | | |
| mean | S | 1-24 | 0.874 | | |
| | D | 1-24 | 0.797 | 0.450 | YES |

Name=Sequence SP='YES' Cleavage site between pos. 24 and 25: LEA-VP D=0.797 D-cutoff=0.450 Networks=SignalP-noTM

[Fig. 1e]

Procollagen C-endopeptidase enhancer 1

# SignalP-4.1 euk predictions
> Sequence

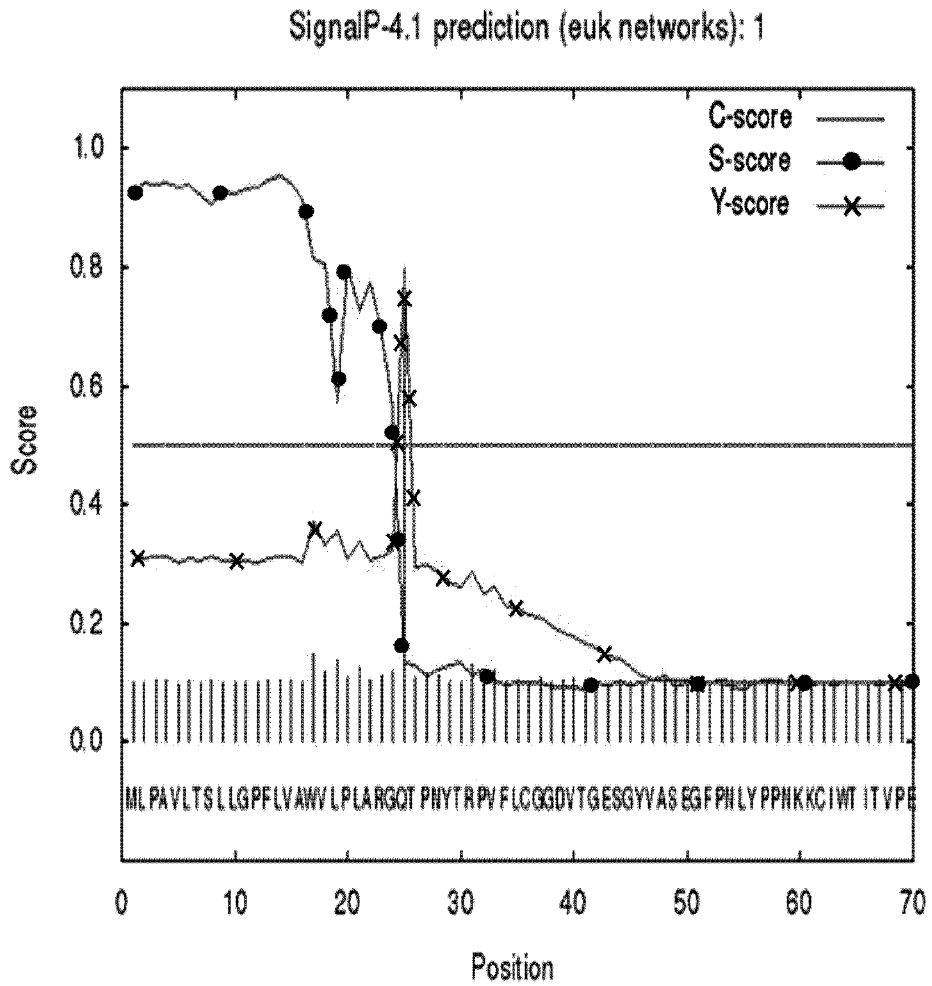


| # Measure | | Position | Value | Cutoff | signal peptide? |
|---|---|---|---|---|---|
| max. | C | 25 | 0.740 | | |
| max. | Y | 25 | 0.795 | | |
| max. | S | 14 | 0.955 | | |
| mean | S | 1-24 | 0.861 | | |
| | D | 1-24 | 0.831 | 0.450 | YES |

Name=Sequence SP='YES' Cleavage site between pos. 24 and 25: ARG-QT D=0.831 D-cutoff=0.450 Networks=SignalP-noTM

[Fig. 1f]

## C-C motif chemokine

# SignalP-4.1 euk predictions
> Sequence

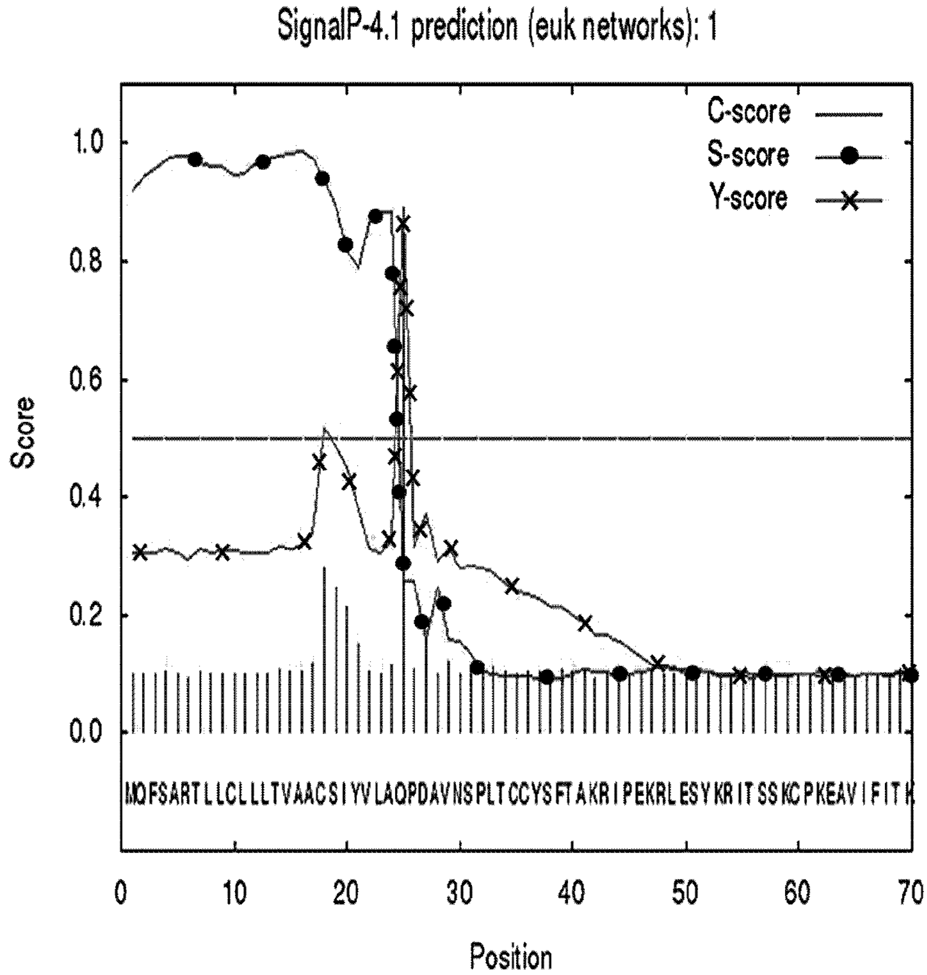


| # Measure | | Position | Value | Cutoff | signal peptide? |
|---|---|---|---|---|---|
| max. | C | 25 | 0.848 | | |
| max. | Y | 25 | 0.891 | | |
| max. | S | 16 | 0.986 | | |
| mean | S | 1-24 | 0.936 | | |
| | D | 1-24 | 0.916 | 0.450 | YES |

Name=Sequence SP='YES' Cleavage site between pos. 24 and 25: VLA-QP D=0.916 D-cutoff=0.450 Networks=SignalP-noTM

[Fig. 1g]

Lipoprotein lipase

# SignalP-4.1 euk predictions

> Sequence

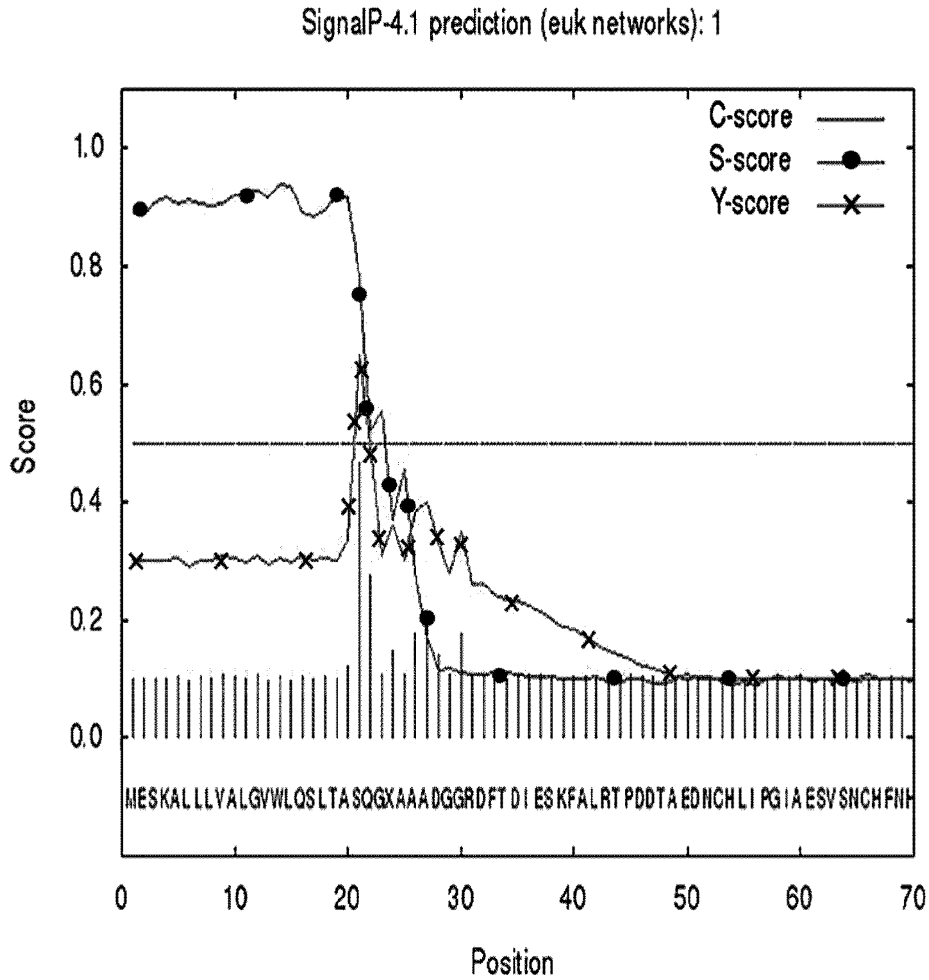


| # Measure | | Position | Value | Cutoff | signal peptide? |
|---|---|---|---|---|---|
| max. | C | 21 | 0.467 | | |
| max. | Y | 21 | 0.651 | | |
| max. | S | 14 | 0.939 | | |
| mean | S | 1-20 | 0.911 | | |
| | D | 1-20 | 0.791 | 0.450 | YES |

Name=Sequence SP='YES' Cleavage site between pos. 20 and 21: LTA-SQ D=0.791 D-cutoff=0.450 Networks=SignalP-noTM

[Fig. 1h]

Nidogen-1

# SignalP-4.1 euk predictions

> Sequence

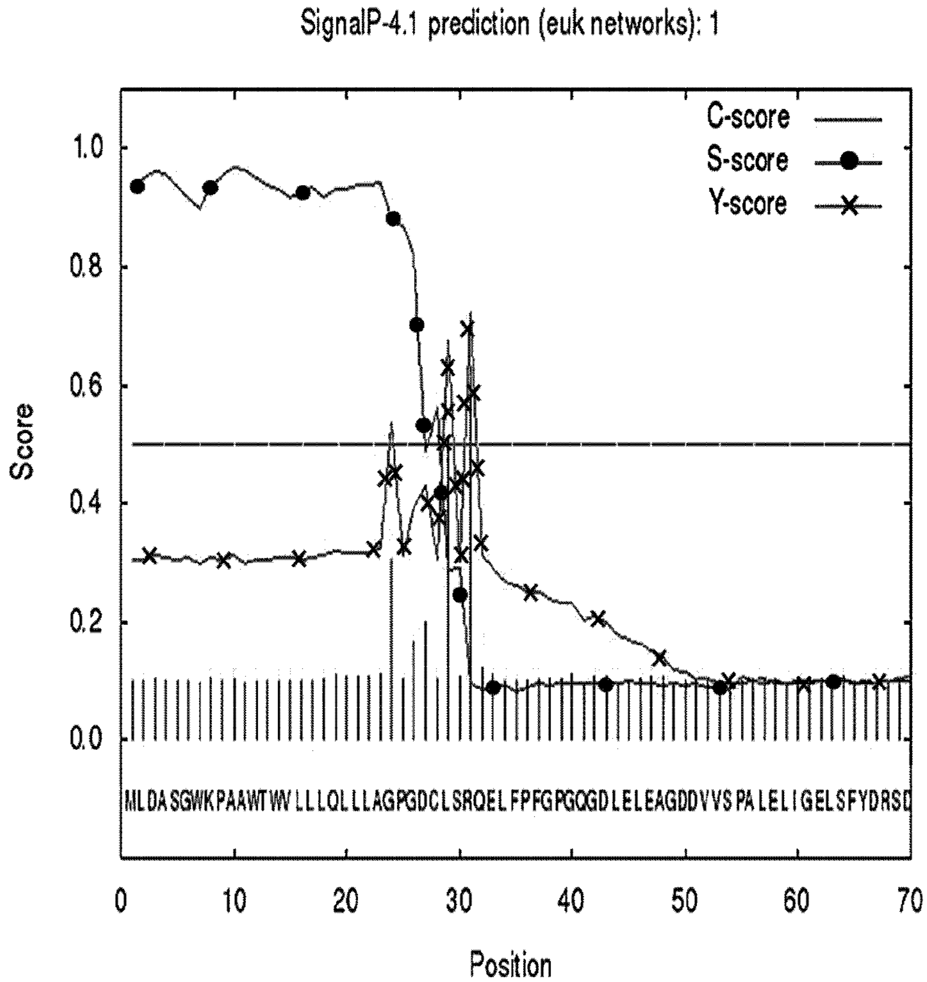


| # Measure | | Position | Value | Cutoff | signal peptide? |
|---|---|---|---|---|---|
| max. | C | 31 | 0.630 | | |
| max. | Y | 31 | 0.723 | | |
| max. | S | 10 | 0.969 | | |
| mean | S | 1-30 | 0.859 | | |
| | D | 1-30 | 0.796 | 0.450 | YES |

Name=Sequence SP='YES' Cleavage site between pos. 30 and 31: CLS-RQ D=0.796 D-cutoff=0.450 Networks=SignalP-noTM

[Fig. 1i]

## Pigment epithelium-derived factor

# SignalP-4.1 euk predictions
> Sequence

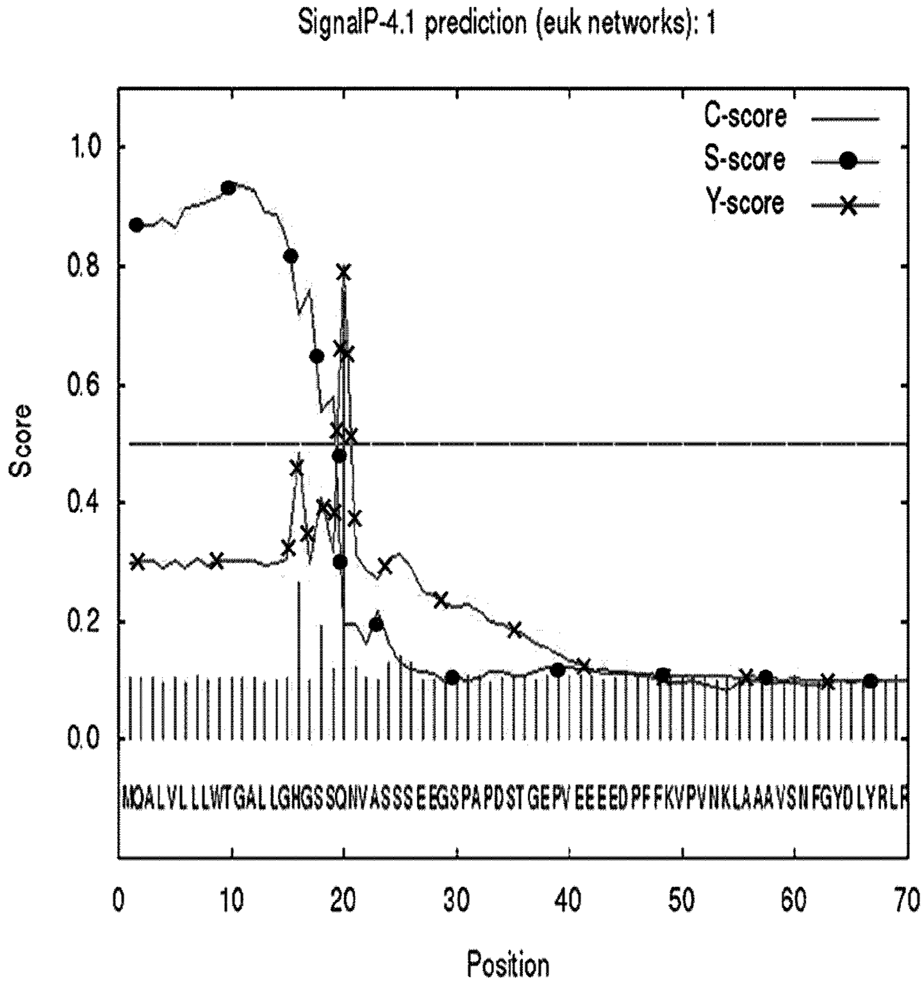


| # Measure | | Position | Value | Cutoff | signal peptide? |
|---|---|---|---|---|---|
| max. | C | 20 | 0.754 | | |
| max. | Y | 20 | 0.799 | | |
| max. | S | 10 | 0.938 | | |
| mean | S | 1-19 | 0.843 | | |
| | D | 1-19 | 0.823 | 0.450 | YES |

Name=Sequence SP='YES' Cleavage site between pos. 19 and 20: GSS-QN D=0.823 D-cutoff=0.450 Networks=SignalP-noTM

[Fig. 1j]

Protein disulfide-isomerase

# SignalP-4.1 euk predictions

> Sequence

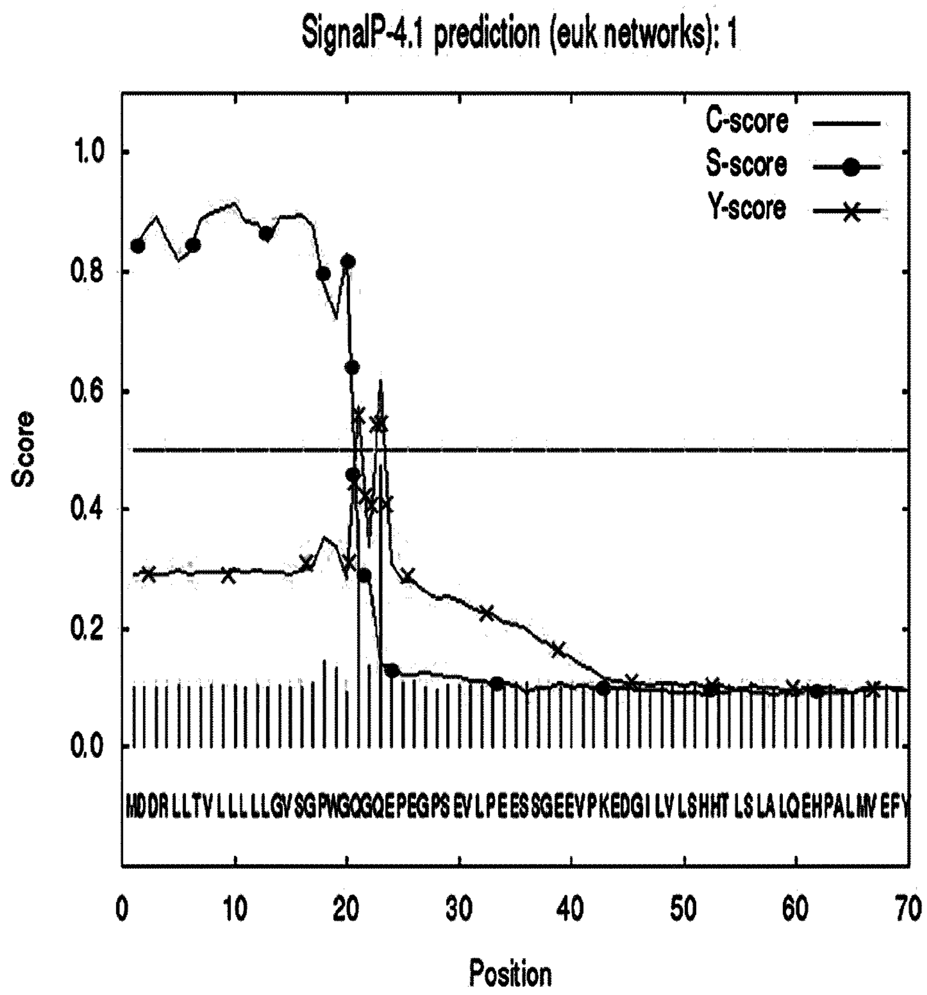


| # Measure | | Position | Value | Cutoff | signal peptide? |
|---|---|---|---|---|---|
| max. | C | 23 | 0.472 | | |
| max. | Y | 23 | 0.618 | | |
| max. | S | 10 | 0.914 | | |
| mean | S | 1-22 | 0.808 | | |
| | D | 1-22 | 0.721 | 0.450 | YES |

Name=Sequence SP='YES' Cleavage site between pos. 22 and 23: GQG-QE D=0.721 D-cutoff=0.450 Networks=SignalP-noTM

[Fig. 2]

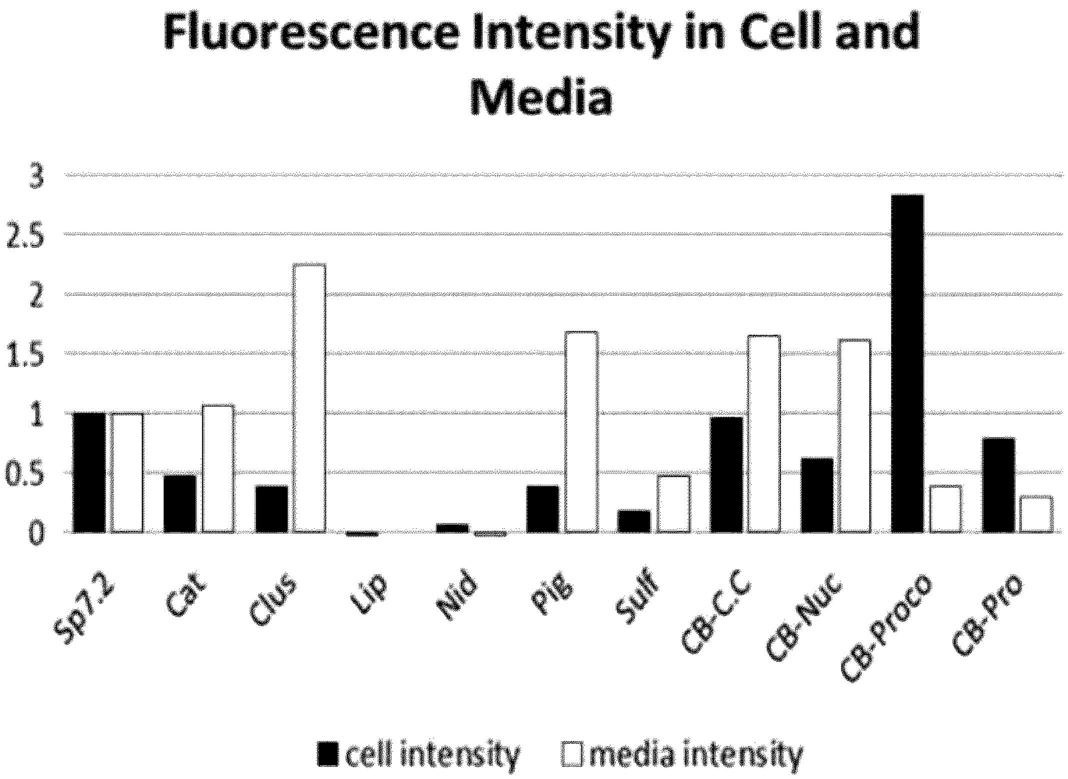
Fluorescence Intensity in Cell and Media
3
2.5
2
1.5
1
0.5
0
Sp7.2
Cat
Clus
Lip
Nid
Pig
Sulf
CB-C.C
CB-Nuc
CB-Proco
CB-Pro
cell intensity
media intensity

[Fig. 3]

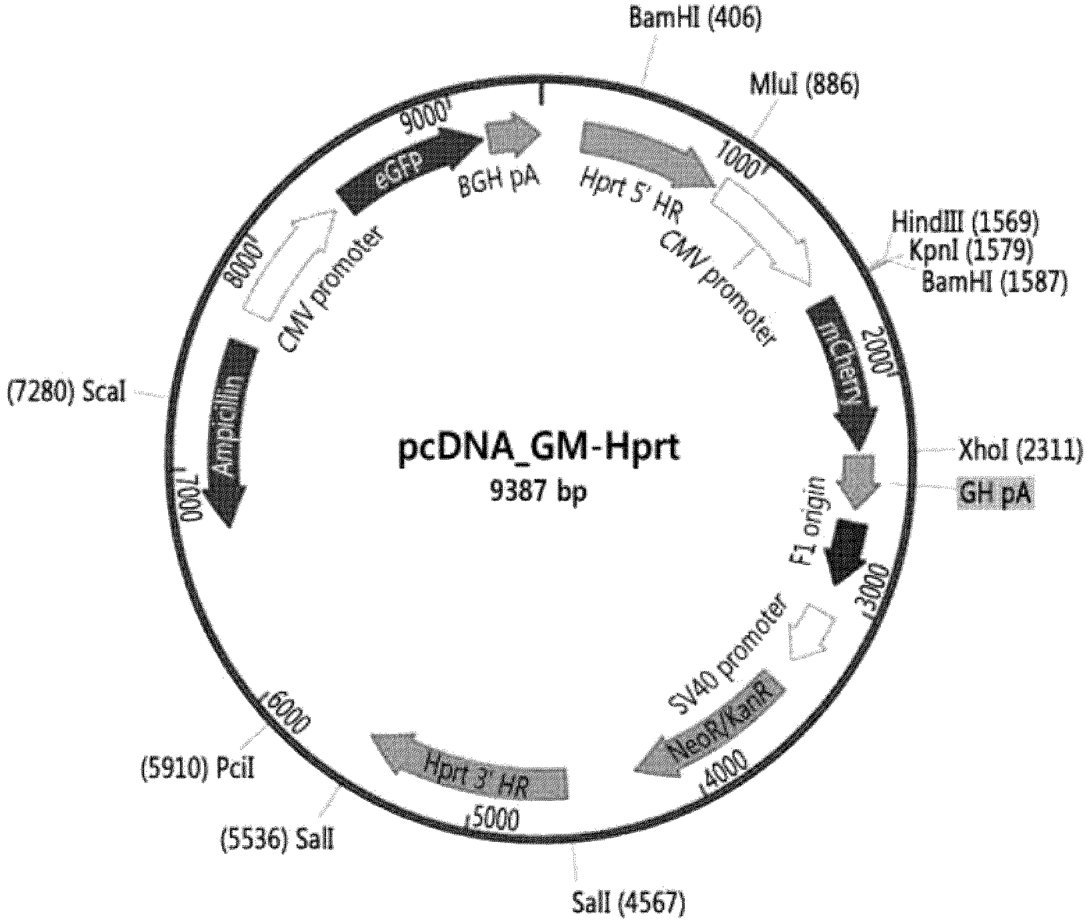
BamHI (406)
MluI (886)
HindIII (1569)
KpnI (1579)
BamHI (1587)
XhoI (2311)
GH pA
SalI (4567)
(5536) SalI
(5910) PciI
(7280) ScaI
pcDNA_GM-Hprt
9387 bp
eGFP
BGH pA
Hprt 5' HR
CMV promoter
CMV promoter
mCherry
F1 origin
SV40 promoter
NeoR/KanR
Hprt 3' HR
Ampicillin
1000
2000
3000
4000
5000
6000
7000
8000
9000

[Fig. 4]

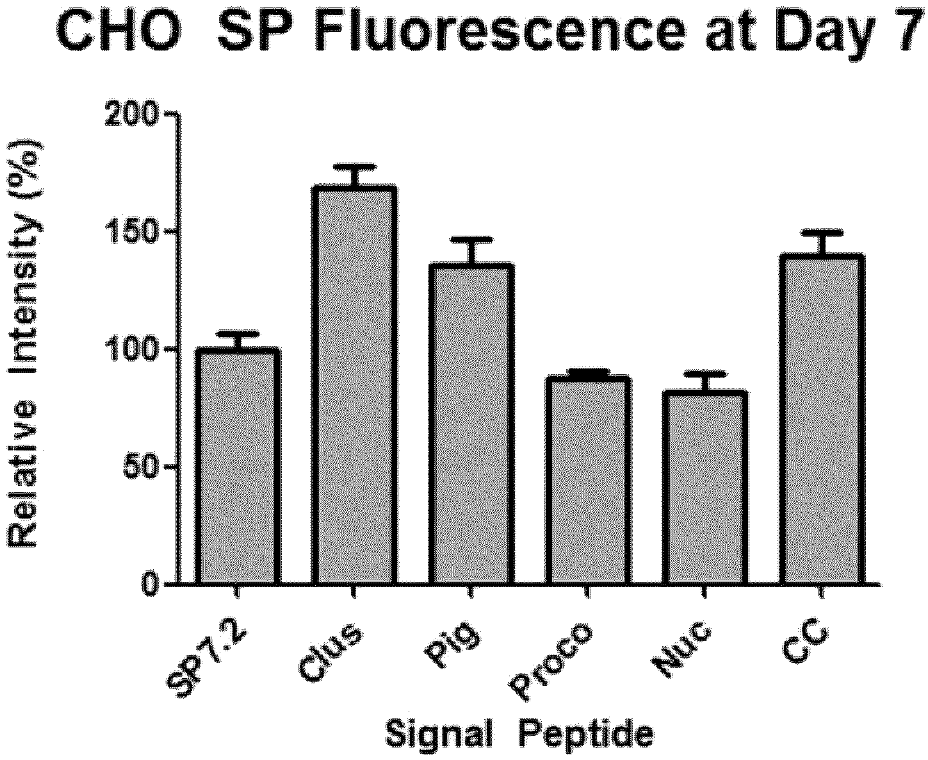
CHO SP Fluorescence at Day 7
Relative Intensity (%)
200
150
100
50
0
SP7.2
Clus
Pig
Proco
Nuc
CC
Signal Peptide

[Fig. 5a]

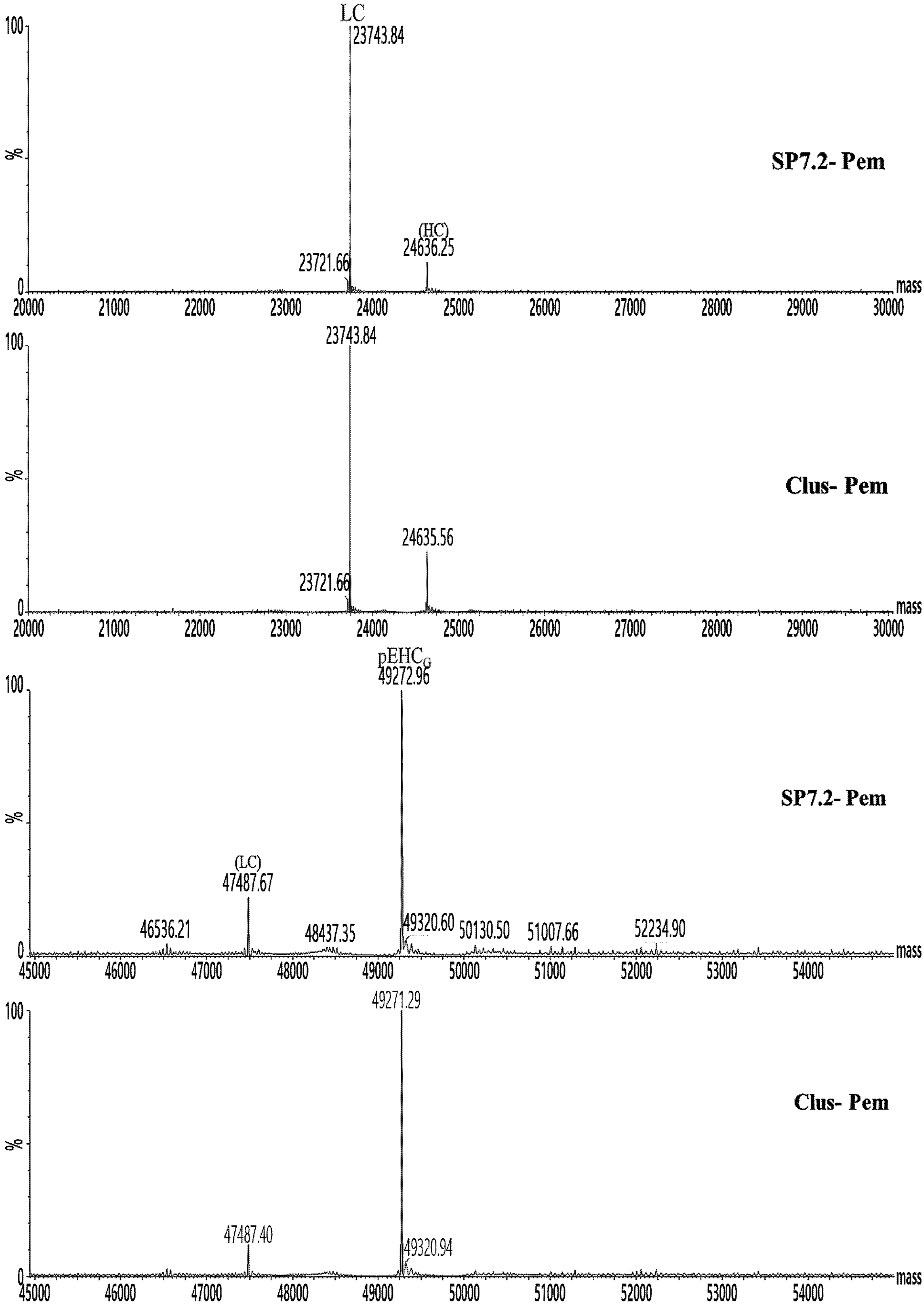
LC
23743.84
SP7.2- Pem
(HC)
24636.25
23721.66
23743.84
Clus- Pem
24635.56
23721.66
pEHC$_G$
49272.96
SP7.2- Pem
(LC)
47487.67
46536.21
48437.35
49320.60
50130.50
51007.66
52234.90
49271.29
Clus- Pem
47487.40
49320.94
100
%
0
mass

[Fig. 5b]

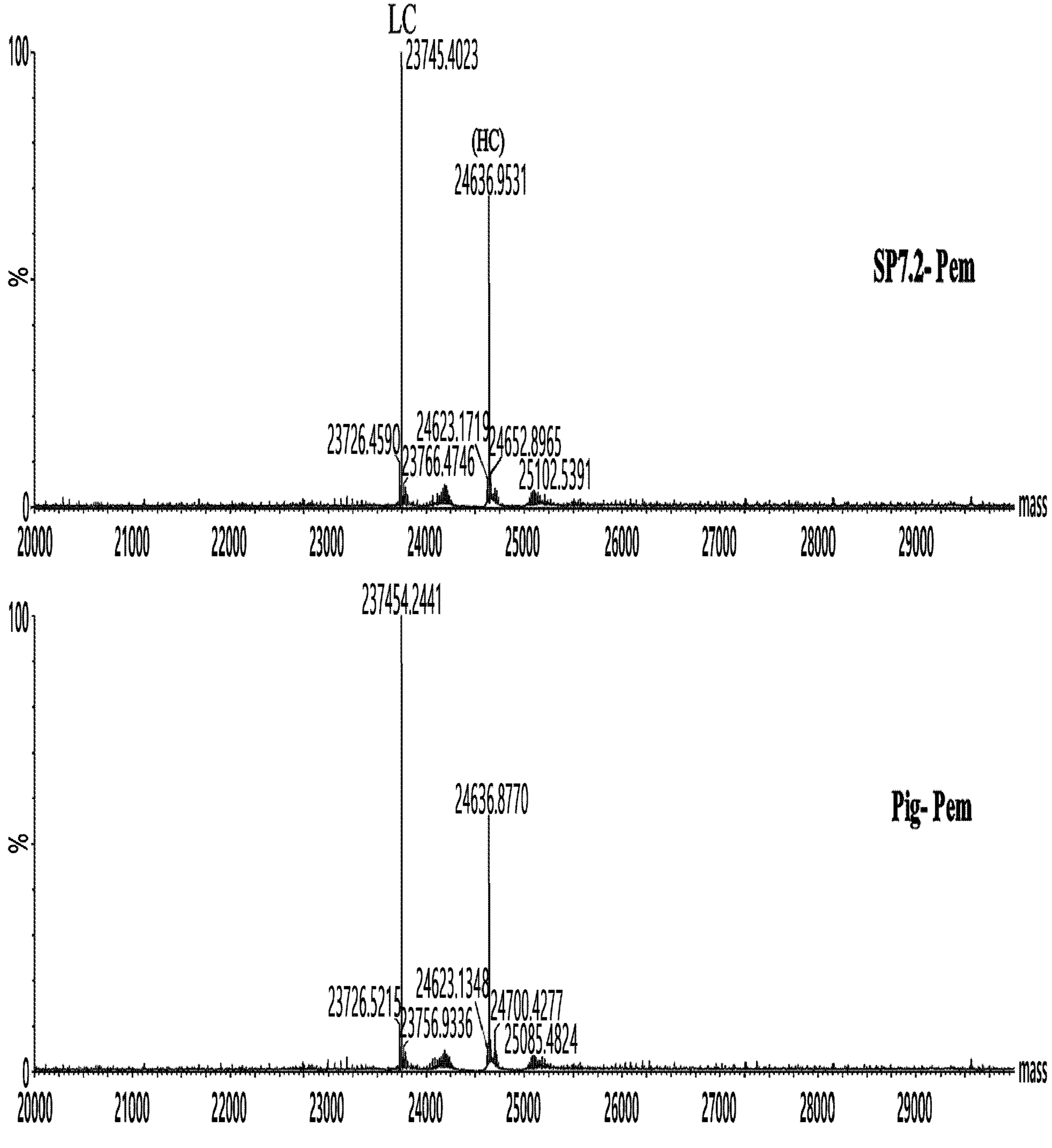
LC
23745.4023
(HC)
24636.9531
SP7.2- Pem
23726.4590
24623.1719
24652.8965
23766.4746
25102.5391
100
%
0
mass
20000
21000
22000
23000
24000
25000
26000
27000
28000
29000
237454.2441
24636.8770
Pig- Pem
23726.5215
24623.1348
24700.4277
23756.9336
25085.4824

[Fig. 5c]

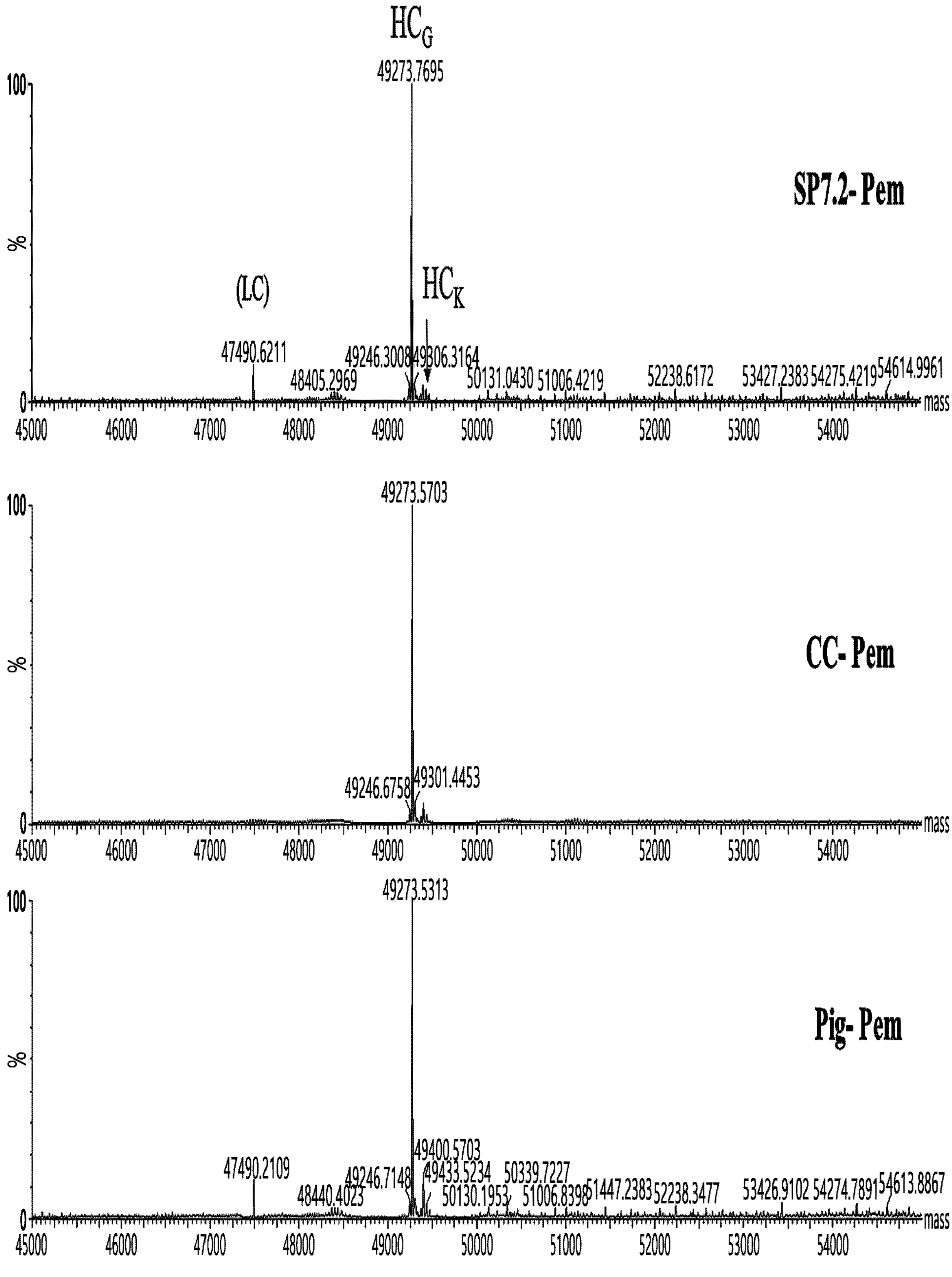
HCG
49273.7695
SP7.2- Pem
(LC)
HCK
47490.6211
48405.2969
49246.3008
49306.3164
50131.0430
51006.4219
52238.6172
53427.2383
54275.4219
54614.9961
mass
49273.5703
CC- Pem
49246.6758
49301.4453
49273.5313
Pig- Pem
47490.2109
48440.4023
49246.7148
49400.5703
49433.5234
50130.1953
50339.7227
51006.8398
51447.2383
52238.3477
53426.9102
54274.7891
54613.8867
%
100
0
45000
46000
47000
48000
49000
50000
51000
52000
53000
54000

## INTERNATIONAL SEARCH REPORT

International application No.
**PCT/KR2020/017413**

**A. CLASSIFICATION OF SUBJECT MATTER**

**C07K 7/08**(2006.01)i; **C07K 14/52**(2006.01)i; **C07K 14/47**(2006.01)i; **C12N 9/02**(2006.01)i; **C12N 9/18**(2006.01)i; **C12N 9/90**(2006.01)i

According to International Patent Classification (IPC) or to both national classification and IPC

**B. FIELDS SEARCHED**

Minimum documentation searched (classification system followed by classification symbols)

C07K 7/08(2006.01); C07K 14/47(2006.01); C12N 15/11(2006.01); C12N 15/12(2006.01); C12N 15/63(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 단백질 분비 인자(protein secretion factor), CHO 세포(CHO cells), 발현 벡터(expression vector)

**C. DOCUMENTS CONSIDERED TO BE RELEVANT**

| Category* | Citation of document, with indication, where appropriate, of the relevant passages | Relevant to claim No. |
|---|---|---|
| A | NCBI Reference Sequence: XP_005075331.1 (22 May 2017). See DEFINITION; and the sequences. | 1-19 |
| A | NCBI. GenBank accession no. ERE67518.1 (22 March 2015). See DEFINITION; and the sequences. | 1-19 |
| A | NCBI. GenBank accession no. ERE80278.1 (22 March 2015). See DEFINITION; and the sequences. | 1-19 |
| A | KR 10-2015-0125402 A (LG LIFE SCIENCES LTD.) 09 November 2015 (2015-11-09) See entire document. | 1-19 |
| A | KR 10-2013-0141001 A (KOREA RESEARCH INSTITUTE OF STANDARDS AND SCIENCE) 26 December 2013 (2013-12-26) See entire document. | 1-19 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 March 2021** | **25 March 2021** |
| Name and mailing address of the ISA/KR<br>**Korean Intellectual Property Office**<br>**Government Complex-Daejeon Building 4, 189 Cheongsa-ro, Seo-gu, Daejeon 35208** | Authorized officer |
| Facsimile No. **+82-42-481-8578** | Telephone No. |

Form PCT/ISA/210 (second sheet) (July 2019)

**INTERNATIONAL SEARCH REPORT**

International application No.
**PCT/KR2020/017413**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2020/017413**

| Patent document cited in search report | Publication date (day/month/year) | Patent family member(s) | Publication date (day/month/year) |
|---|---|---|---|
| KR 10-2015-0125402 A | 09 November 2015 | AR 100255 A1 | 21 September 2016 |
| | | BR 112016024895 A2 | 24 October 2017 |
| | | CN 106255700 A | 21 December 2016 |
| | | EP 3137493 A1 | 08 March 2017 |
| | | EP 3137493 B1 | 04 September 2019 |
| | | JP 2017-513511 A | 01 June 2017 |
| | | JP 2019-006823 A | 17 January 2019 |
| | | JP 6471175 B2 | 13 February 2019 |
| | | KR 10-2092225 B1 | 23 March 2020 |
| | | MX 2016013849 A | 02 February 2017 |
| | | TW 201625665 A | 16 July 2016 |
| | | TW I708780 B | 01 November 2020 |
| | | US 10407475 B2 | 10 September 2019 |
| | | US 2017-0044224 A1 | 16 February 2017 |
| | | US 2020-0255485 A1 | 13 August 2020 |
| | | WO 2015-167278 A1 | 05 November 2015 |
| KR 10-2013-0141001 A | 26 December 2013 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101038126
- KR 1020150125402 A
- KR 101038126 B1

### Non-patent literature cited in the description


- **PEARSON et al.** *Proc. Natl. Acad. Sci. USA,* 1988, vol. 85, 2444
- **NEEDLEMAN ; WUNSCH.** *J. Mol. Biol.,* 1970, vol. 48, 443-453
- **RICE et al.** *Trends Genet.,* 2000, vol. 16, 276-277
- **DEVEREUX, J. et al.** *Nucleic Acids Research,* 1984, vol. 12, 387
- **ATSCHUL, [S.] [F.** *J MOLEC BIOL,* 1990, vol. 215, 403
- Guide to Huge Computers. Academic Press, 1994
- **CARILLO.** *SIAM J Applied Math,* 1988, vol. 48, 1073
- **NEEDLEMAN et al.** *J Mol Biol.,* 1970, vol. 48, 443
- **SMITH ; WATERMAN.** *Adv. Appl. Math,* 1981, vol. 2, 482
- **GRIBSKOV et al.** *Nucl. Acids Res,* 1986, vol. 14, 6745
- Atlas of Protein Sequence and Structure. National Biomedical Research Foundation, 1979, 353-358
- **J.S LEE et al.** Site-Specific integration in CHO cells mediated by CRISPR/Cas9 and homology-directed DNA repair pathway. *Sci. Rep.,* 2015, vol. 5

<110> LG CHEM, LTD.

<120> CHO cells-derived protein secretory factors and expression vectors comprising the same

<130> OPA20173

<150> KR 10-2019-0158447
<151> 2019-12-02

<160> 59

<170> KoPatentIn 3.0

<210> 1
<211> 17
<212> PRT
<213> Unknown

<220>
<223> Signal Peptide of Cathepsin B (Cat)

<400> 1
```
Met Trp Trp Ser Leu Ile Pro Leu Ser Cys Leu Leu Ala Leu Ala Ser
  1               5                  10                  15

Ala
```

<210> 2
<211> 24
<212> PRT
<213> Unknown

<220>
<223> Signal Peptide of C-C motif chemokine (CC)

<400> 2
```
Met Gln Phe Ser Ala Arg Thr Leu Leu Cys Leu Leu Leu Thr Val Ala
  1               5                  10                  15

Ala Cys Ser Ile Tyr Val Leu Ala
             20
```

<210> 3
<211> 24
<212> PRT
<213> Unknown

<220>
<223> Signal Peptide of Nucleobindin-2 (Nuc)

<400> 3
```
Met Arg Trp Lys Ile Ile Gln Leu Gln Tyr Cys Phe Leu Leu Val Pro
  1               5                  10                  15

Cys Met Leu Thr Ala Leu Glu Ala
             20
```

<210> 4
<211> 21
<212> PRT
<213> Unknown

<220>
<223> Signal Peptide of Clusterin (Clus)

<400> 4

```
Met Lys Ile Leu Leu Leu Cys Val Gly Leu Leu Leu Thr Trp Asp Asn
1               5                   10                  15

Gly Met Val Leu Gly
            20
```

<210> 5
<211> 19
<212> PRT
<213> Unknown

<220>
<223> Signal Peptide of Pigment epithelium-derived factor (Pig)

<400> 5

```
Met Gln Ala Leu Val Leu Leu Leu Trp Thr Gly Ala Leu Leu Gly His
1               5                   10                  15

Gly Ser Ser
```

<210> 6
<211> 24
<212> PRT
<213> Unknown

<220>
<223> Signal Peptide of Procollagen C-endopeptidase enhancer 1 (Proco)

<400> 6

```
Met Leu Pro Ala Val Leu Thr Ser Leu Leu Gly Pro Phe Leu Val Ala
1               5                   10                  15

Trp Val Leu Pro Leu Ala Arg Gly
            20
```

<210> 7
<211> 31
<212> PRT
<213> Unknown

<220>
<223> Signal Peptide of Sulfhydryl oxidase (Sulf)

<400> 7

Met Arg Arg Cys Gly Arg His Ser Gly Ser Pro Ser Gln Met Leu Leu
1 5 10 15

Leu Leu Leu Pro Pro Leu Leu Leu Ala Val Pro Gly Ala Gly Ala
20 25 30

<210> 8
<211> 20
<212> PRT
<213> Unknown

<220>
<223> Signal Peptide of Lipoprotein lipase (Lip)

<400> 8
Met Glu Ser Lys Ala Leu Leu Leu Val Ala Leu Gly Val Trp Leu Gln
1 5 10 15

Ser Leu Thr Ala
20

<210> 9
<211> 30
<212> PRT
<213> Unknown

<220>
<223> Signal Peptide of Nidogen-1 (Nid)

<400> 9
Met Leu Asp Ala Ser Gly Trp Lys Pro Ala Ala Trp Thr Trp Val Leu
1 5 10 15

Leu Leu Gln Leu Leu Leu Ala Gly Pro Gly Asp Cys Leu Ser
20 25 30

<210> 10
<211> 22
<212> PRT
<213> Unknown

<220>
<223> Signal Peptide of Protein disulfide-isomerase (Pro)

<400> 10
Met Asp Asp Arg Leu Leu Thr Val Leu Leu Leu Leu Leu Gly Val Ser
1 5 10 15

Gly Pro Trp Gly Gln Gly
20

<210> 11
<211> 51
<212> DNA
<213> Unknown

<220>
<223> gene of Signal Peptide of Cathepsin B (Cat)

<400> 11
atgtggtggt ccttgattcc gctctcttgc ctgctggcac tggcaagtgc c 51

<210> 12
<211> 72
<212> DNA
<213> Unknown

<220>
<223> gene of Signal Peptide of C-C motif chemokine (CC)

<400> 12
atgcagttct ccgcaagaac gcttctgtgc ctgctactca cagttgctgc ctgtagcatc 60

tatgtgctgg cc 72

<210> 13
<211> 72
<212> DNA
<213> Unknown

<220>
<223> gene of Signal Peptide of Nucleobindin-2 (Nuc)

<400> 13
atgaggtgga agatcatcca gctacagtac tgttttcttt tggtcccgtg catgcttact 60

gctctggaag ct 72

<210> 14
<211> 63
<212> DNA
<213> Unknown

<220>
<223> gene of Signal Peptide of Clusterin (Clus)

<400> 14
atgaagattc tcctgttgtg cgtggggctg ctgctgacct gggacaatgg catggtcctg 60

gga 63

<210> 15
<211> 57
<212> DNA
<213> Unknown

<220>
<223> gene of Signal Peptide of Pigment epithelium-derived factor (Pig)

<400> 15
atgcaggccc tggtgctact cctctggaca ggagccctgc ttgggcatgg cagcagc 57

<210> 16
<211> 72
<212> DNA
<213> Unknown

<220>
<223> gene of Signal Peptide of Procollagen C-endopeptidase enhancer 1 (Proco)

<400> 16
atgctgcctg ctgtcctaac ctccctcctg ggccattcc ttgtggcctg ggtactgcct 60

cttgcccgag gc 72

<210> 17
<211> 93
<212> DNA
<213> Unknown

<220>
<223> gene of Signal Peptide of Sulfhydryl oxidase (Sulf)

<400> 17
atgaggaggt gcggccgcca ctcggggtcg ccgtcgcaga tgctactgct gctgctgccg 60

ccgctgctgc tcgcggtgcc cggcgctggc gcg 93

<210> 18
<211> 60
<212> DNA
<213> Unknown

<220>
<223> gene of Signal Peptide of Lipoprotein lipase (Lip)

<400> 18
atggagagca aagccctgct cctggtggct ctgggagtgt ggctccagag tttgaccgcc 60

60

<210> 19
<211> 90
<212> DNA
<213> Unknown

<220>
<223> gene of Signal Peptide of Nidogen-1 (Nid)

<400> 19
atgctggacg cgagcggctg gaagcccgcg gcgtggacat gggtgctgct gctgcagcta 60

ttgctggcgg ggcccggaga ctgcctgagc 90

<210> 20
<211> 66
<212> DNA
<213> Unknown

<220>
<223> gene of Signal Peptide of Protein disulfide-isomerase (Pro)

<400> 20
atggatgatc ggctcctgac agtgttgctg ctcctgctgg gtgtctcagg cccatgggga 60

caggga 66

<210> 21
<211> 339
<212> PRT
<213> Unknown

<220>
<223> Cathepsin B (Cat)

<400> 21
```
Met Trp Trp Ser Leu Ile Pro Leu Ser Cys Leu Leu Ala Leu Ala Ser
1               5                   10                  15

Ala His Asn Lys Pro Ser Phe His Pro Leu Ser Asp Asp Leu Ile Asn
            20                  25                  30

Tyr Ile Asn Lys Arg Asn Thr Thr Trp Gln Ala Gly Arg Asn Phe His
        35                  40                  45

Asn Val Asp Ile Ser Tyr Leu Lys Arg Leu Cys Gly Thr Ile Met Gly
    50                  55                  60

Gly Pro Lys Leu Pro Glu Arg Val Ala Phe Ala Glu Asp Met Glu Leu
65                  70                  75                  80

Pro Glu Asn Phe Asp Ala Arg Glu Gln Trp Ser Asn Cys Pro Thr Ile
                85                  90                  95

Lys Gln Ile Arg Asp Gln Gly Ser Cys Gly Ser Cys Trp Ala Phe Gly
            100                 105                 110

Ala Val Gly Ala Met Ser Asp Arg Leu Cys Ile His Thr Asn Gly His
        115                 120                 125

Val Asn Val Glu Val Ser Ala Glu Asp Leu Leu Thr Cys Cys Gly Ser
    130                 135                 140

Gln Cys Gly Asp Gly Cys Asn Gly Gly Tyr Pro Ser Gly Ala Trp Asn
145                 150                 155                 160

Phe Trp Ile Lys Lys Gly Leu Val Ser Gly Gly Leu Tyr Asn Ser His
                165                 170                 175

Val Gly Cys Leu Pro Tyr Thr Ile Pro Pro Cys Glu His His Val Asn
            180                 185                 190
```

Gly Ser Arg Pro Gln Cys Thr Gly Glu Gly Asp Thr Pro Lys Cys Thr
195 200 205

Lys Ser Cys Glu Ala Gly Tyr Ser Pro Ser Tyr Lys Glu Asp Lys His
210 215 220

Tyr Gly Tyr Thr Ser Tyr Ser Val Ser Asn Asn Glu Lys Glu Ile Met
225 230 235 240

Ala Glu Ile Tyr Lys Asn Gly Pro Val Glu Gly Ala Phe Thr Val Phe
245 250 255

Ser Asp Phe Leu Thr Tyr Lys Ser Gly Val Tyr Lys His Glu Ala Gly
260 265 270

Asp Ile Met Gly Gly His Ala Ile Arg Ile Leu Gly Trp Gly Val Glu
275 280 285

Asn Ser Val Pro Tyr Trp Leu Val Ala Asn Ser Trp Asn Val Asp Trp
290 295 300

Gly Asp Asn Gly Leu Phe Lys Ile Leu Arg Gly Glu Asp His Cys Gly
305 310 315 320

Ile Glu Ser Glu Ile Val Ala Gly Ile Pro Arg Thr Asp Leu Tyr Trp
325 330 335

Gly Arg Phe

<210> 22
<211> 143
<212> PRT
<213> Unknown

<220>
<223> C-C motif chemokine (CC)

<400> 22
Met Gln Phe Ser Ala Arg Thr Leu Leu Cys Leu Leu Leu Thr Val Ala
1 5 10 15

Ala Cys Ser Ile Tyr Val Leu Ala Gln Pro Asp Ala Val Asn Ser Pro
20 25 30

Leu Thr Cys Cys Tyr Ser Phe Thr Ala Lys Arg Ile Pro Glu Lys Arg
35 40 45

Leu Glu Ser Tyr Lys Arg Ile Thr Ser Ser Lys Cys Pro Lys Glu Ala
50 55 60

Val Ile Phe Ile Thr Lys Leu Lys Arg Glu Ile Cys Ala Asp Pro Lys
65 70 75 80

Gln Asp Trp Val Gln Thr Tyr Thr Lys Lys Leu Asp Gln Ser Gln Ala
85 90 95

Lys Ser Glu Ala Ala Thr Val Tyr Lys Thr Ala Pro Leu Asn Ala Asn
100 105 110

Leu Thr His Glu Ser Ala Val Asn Ala Ser Thr Thr Ala Phe Pro Thr

115 120 125

Thr Asp Leu Arg Thr Ser Val Arg Val Thr Ser Met Thr Val Asn
130 135 140

<210> 23
<211> 420
<212> PRT
<213> Unknown

<220>
<223> Nucleobindin-2 (Nuc)

<400> 23
Met Arg Trp Lys Ile Ile Gln Leu Gln Tyr Cys Phe Leu Leu Val Pro
1 5 10 15

Cys Met Leu Thr Ala Leu Glu Ala Val Pro Ile Asp Val Asp Lys Thr
20 25 30

Lys Val His Asn Thr Glu Pro Val Glu Ser Ala Arg Ile Asp Pro Pro
35 40 45

Asp Thr Gly Leu Tyr Tyr Asp Glu Tyr Leu Lys Gln Val Ile Asp Val
50 55 60

Leu Glu Thr Asp Gln His Phe Arg Glu Lys Leu Gln Lys Ala Asp Ile
65 70 75 80

Glu Glu Ile Arg Ser Gly Arg Leu Ser Lys Glu Leu Asp Leu Val Ser
85 90 95

His His Val Arg Thr Lys Leu Asp Glu Leu Lys Arg Gln Glu Val Ala
100 105 110

Arg Leu Arg Met Leu Ile Lys Ala Lys Leu Asp Ser Leu Gln Asp Thr
115 120 125

Gly Met Asn His His Leu Leu Leu Lys Gln Phe Glu His Leu Asn His
130 135 140

Gln Asn Pro Asp Thr Phe Glu Ser Ser Asp Leu Asp Met Leu Ile Lys
145 150 155 160

Ala Ala Thr Ala Asp Leu Glu Gln Tyr Asp Arg Thr Arg His Glu Glu
165 170 175

Phe Lys Lys Tyr Glu Met Met Lys Glu His Glu Arg Arg Glu Tyr Leu
180 185 190

Lys Thr Leu Asn Glu Glu Lys Arg Lys Glu Glu Glu Ser Lys Phe Glu
195 200 205

Glu Met Lys Arg Lys His Glu Asn His Pro Lys Val Asn His Pro Gly
210 215 220

Ser Lys Asp Gln Leu Lys Glu Val Trp Glu Glu Ala Asp Gly Leu Asp
225 230 235 240

Pro Asn Asp Phe Asp Pro Lys Thr Phe Phe Lys Leu His Asp Val Asn
245 250 255

Asn Asp Gly Phe Leu Asp Glu Gln Glu Leu Glu Ala Leu Phe Thr Arg
260 265 270

Glu Leu Glu Lys Val Tyr Asp Pro Arg Asn Ala Glu Asp Asp Met Ile
275 280 285

Glu Met Glu Glu Glu Arg Leu Arg Met Arg Glu His Val Met Asn Glu
290 295 300

Ile Asp Asn Asn Lys Asp Arg Leu Val Thr Leu Glu Glu Phe Leu Arg
305 310 315 320

Ala Thr Glu Lys Lys Glu Phe Leu Glu Pro Asp Ser Trp Glu Thr Leu
325 330 335

Gly Gln Gln Gln Leu Phe Thr Glu Glu Glu Leu Lys Glu Tyr Glu Ser
340 345 350

Ile Ile Ala Met Gln Glu Asn Glu Leu Lys Lys Arg Ala Asp Glu Leu
355 360 365

Gln Lys Gln Lys Glu Glu Leu Gln Arg Gln His Asp His Leu Glu Ala
370 375 380

Gln Lys Gln Glu Tyr Gln Gln Ala Val Gln Gln Leu Glu His Lys Lys
385 390 395 400

Phe Gln Gln Gly Ile Ala Pro Ser Gly Pro Ala Gly Glu Leu Glu Phe
405 410 415

Lys Pro Arg Met
420

<210> 24
<211> 447
<212> PRT
<213> Unknown

<220>
<223> Clusterin (Clus)

<400> 24
Met Lys Ile Leu Leu Leu Cys Val Gly Leu Leu Leu Thr Trp Asp Asn
1 5 10 15

Gly Met Val Leu Gly Glu Gln Glu Val Ser Asp Asn Glu Leu Lys Glu
20 25 30

Met Ser Thr Gln Gly Ser Arg Tyr Ile Asn Lys Glu Ile Gln Asn Ala
35 40 45

Val Gln Gly Val Lys Gln Ile Lys Thr Leu Ile Glu Lys Thr Asn Glu
50 55 60

Glu Arg Lys Ser Leu Leu Asn Ser Leu Glu Glu Ala Lys Lys Lys Lys
65 70 75 80

Glu Asp Ala Leu Asp Asp Thr Arg Asp Thr Glu Met Lys Leu Lys Ala

85 90 95

Phe Pro Glu Val Cys Asn Glu Thr Met Met Ala Leu Trp Glu Glu Cys
100 105 110

Lys Pro Cys Leu Lys Gln Thr Cys Met Lys Phe Tyr Ala Arg Val Cys
115 120 125

Arg Ser Gly Ser Gly Leu Val Gly Arg Gln Leu Glu Glu Phe Leu Asn
130 135 140

Gln Ser Ser Pro Phe Tyr Phe Trp Met Asn Gly Asp Arg Ile Asp Ser
145 150 155 160

Leu Met Glu Ser Asp Arg Gln Gln Ser Gln Val Leu Asp Ala Met Gln
165 170 175

Asp Ser Phe Thr Arg Ala Ser Gly Ile Met Asp Met Leu Phe Gln Asp
180 185 190

Arg Phe Phe Thr His Glu Pro Gln Asp Thr His Tyr Phe Ser Pro Phe
195 200 205

Gly Phe Pro His Arg Arg Pro His Phe Leu Tyr Pro Lys Ser Arg Leu
210 215 220

Val Arg Ser Leu Ile Pro Leu Ser His Tyr Gly Pro Pro Ser Phe His
225 230 235 240

Asp Met Phe Gln Pro Phe Leu Glu Met Ile His Gln Ala Gln Gln Ala
245 250 255

Met Asp Val Gln Phe His Arg Pro Ala Phe Gln Phe Pro Asp Lys Gly
260 265 270

Leu Arg Glu Gly Glu Asp Asp Arg Ala Val Cys Lys Glu Ile Arg His
275 280 285

Asn Ser Thr Gly Cys Leu Lys Met Lys Gly Gln Cys Glu Lys Cys Gln
290 295 300

Glu Ile Leu Ser Val Asp Cys Ser Ala Asn Asn Pro Ala Gln Ala His
305 310 315 320

Leu Arg Gln Glu Leu Asn Asp Ser Leu Gln Met Ala Glu Arg Leu Thr
325 330 335

Gln Gln Tyr Asn Glu Leu Leu His Ser Leu Gln Thr Lys Met Leu Asn
340 345 350

Thr Ser Ser Leu Leu Glu Gln Leu Asn Glu Gln Phe Asn Trp Val Ser
355 360 365

Gln Leu Ala Asn Leu Thr Gln Gly Glu Asp Gln Tyr Tyr Leu Arg Val
370 375 380

Ser Thr Val Thr Thr His Ser Asn Asn Ser Glu Glu Pro Ser Arg Val
385 390 395 400

Thr Glu Val Val Val Lys Leu Phe Asp Ser Asp Pro Ile Thr Val Val
405 410 415

Leu Pro Glu Glu Val Ser Lys Asp Asn Pro Lys Phe Met Asp Thr Val

420 425 430

Ala Glu Lys Ala Leu Gln Glu Tyr Arg Lys Lys Ser Arg Ala Glu
435 440 445

<210> 25
<211> 378
<212> PRT
<213> Unknown

<220>
<223> Pigment epithelium-derived factor (Pig)

<400> 25
Met Gln Ala Leu Val Leu Leu Leu Trp Thr Gly Ala Leu Leu Gly His
1 5 10 15

Gly Ser Ser Gln Asn Val Ala Ser Ser Ser Glu Glu Gly Ser Pro Ala
20 25 30

Pro Asp Ser Thr Gly Glu Pro Val Glu Glu Glu Glu Asp Pro Phe Phe
35 40 45

Lys Val Pro Val Asn Lys Leu Ala Ala Ala Val Ser Asn Phe Gly Tyr
50 55 60

Asp Leu Tyr Arg Leu Arg Ser Ser Ala Ser Pro Thr Ala Asn Val Leu
65 70 75 80

Leu Ser Pro Leu Ser Val Ala Thr Ala Leu Ser Ala Leu Ser Leu Gly
85 90 95

Ala Glu Gln Arg Thr Glu Ser Ile Ile His Arg Ala Leu Tyr Tyr Asp
100 105 110

Leu Ile Ser Asn Ser Asp Ile His Ser Thr Tyr Lys Glu Leu Leu Ala
115 120 125

Ser Val Thr Ala Pro Glu Lys Ser Leu Lys Ser Ala Ser Arg Ile Val
130 135 140

Phe Glu Arg Lys Leu Arg Val Arg Ser Ser Phe Val Ala Pro Leu Glu
145 150 155 160

Lys Ser Tyr Gly Thr Arg Pro Arg Ile Leu Thr Gly Asn Pro Arg Ile
165 170 175

Asp Leu Gln Glu Ile Asn Asn Trp Ile Gln Ala Gln Met Lys Gly Lys
180 185 190

Leu Ala Arg Ser Thr Arg Glu Met Pro Ser Ala Ile Ser Ile Leu Leu
195 200 205

Leu Gly Val Ala Tyr Phe Lys Gly Gln Trp Val Thr Lys Phe Asp Ser
210 215 220

Arg Lys Thr Ser Leu Gln Asp Phe His Leu Asp Glu Asp Arg Thr Val
225 230 235 240

```
Lys Val Pro Met Met Ser Glu Pro Lys Ala Ile Leu Arg Tyr Gly Leu
                245                 250                 255

Asp Ser Asp Leu Asn Cys Lys Val Trp Glu His Gly Gly Trp Glu Gly
            260                 265                 270

Ser Glu Arg Gly Arg Val Ser Ser Ile Arg Lys Ser Ile Trp Gly Tyr
        275                 280                 285

Ser Lys Ile His Glu Leu Gln Ser Leu Phe Glu Ser Pro Asp Phe Ser
    290                 295                 300

Lys Ile Thr Gly Lys Pro Val Lys Leu Thr Gln Val Glu His Arg Ala
305                 310                 315                 320

Ala Phe Glu Trp Asn Glu Glu Gly Val Glu Thr Ser Pro Asn Pro Gly
                325                 330                 335

Leu Gln Pro Val Arg Leu Thr Phe Pro Leu Asp Tyr His Leu Asn Gln
            340                 345                 350

Pro Phe Ile Phe Val Leu Arg Asp Thr Asp Thr Gly Ala Leu Leu Phe
        355                 360                 365

Ile Gly Lys Ile Leu Asp Pro Arg Gly Thr
    370                 375
```

<210> 26
<211> 468
<212> PRT
<213> Unknown

<220>
<223> Procollagen C-endopeptidase enhancer 1 (Proco)

<400> 26

```
Met Leu Pro Ala Val Leu Thr Ser Leu Leu Gly Pro Phe Leu Val Ala
1               5                   10                  15

Trp Val Leu Pro Leu Ala Arg Gly Gln Thr Pro Asn Tyr Thr Arg Pro
            20                  25                  30

Val Phe Leu Cys Gly Gly Asp Val Thr Gly Glu Ser Gly Tyr Val Ala
        35                  40                  45

Ser Glu Gly Phe Pro Asn Leu Tyr Pro Pro Asn Lys Lys Cys Ile Trp
    50                  55                  60

Thr Ile Thr Val Pro Glu Gly Gln Thr Val Ser Leu Ser Phe Arg Val
65                  70                  75                  80

Phe Asp Met Glu Leu His Pro Ser Cys Arg Tyr Asp Ala Leu Glu Val
                85                  90                  95

Phe Ala Gly Ser Gly Thr Ser Gly Gln Arg Leu Gly Arg Phe Cys Gly
            100                 105                 110

Thr Phe Arg Pro Ala Pro Val Val Ala Pro Gly Asn Gln Val Thr Leu
        115                 120                 125

Arg Met Thr Thr Asp Glu Gly Thr Gly Gly Arg Gly Phe Leu Leu Trp
```

```
        130                 135                 140

Tyr Ser Gly Arg Ala Thr Ser Gly Thr Glu His Gln Phe Cys Gly Gly
145                 150                 155                 160

Arg Met Glu Lys Ala Gln Gly Thr Leu Thr Thr Pro Asn Trp Pro Glu
                165                 170                 175

Ser Asp Tyr Pro Pro Gly Ile Ser Cys Ser Trp His Ile Ile Ala Pro
            180                 185                 190

Ser Asp Gln Val Ile Met Leu Thr Phe Gly Lys Phe Asp Val Glu Pro
        195                 200                 205

Asp Thr Tyr Cys Arg Tyr Asp Ser Val Ser Val Phe Asn Gly Ala Val
    210                 215                 220

Ser Asp Asp Ser Lys Arg Leu Gly Lys Phe Cys Gly Asp Lys Ala Pro
225                 230                 235                 240

Ser Pro Ile Ser Ser Glu Gly Asn Glu Leu Leu Val Gln Phe Val Ser
                245                 250                 255

Asp Leu Ser Val Thr Ala Asp Gly Phe Ser Ala Ser Tyr Lys Thr Leu
            260                 265                 270

Pro Arg Asp Ala Val Glu Lys Glu Leu Ala Pro Ser Pro Gly Glu Asp
        275                 280                 285

Val Gln Leu Gly Pro Gln Ser Arg Ser Asp Pro Lys Thr Gly Thr Gly
    290                 295                 300

Pro Lys Val Lys Pro Pro Ser Lys Pro Lys Phe Gln Pro Ala Glu Lys
305                 310                 315                 320

Pro Glu Val Ser Pro Asp Thr Gln Glu Thr Pro Val Ala Pro Asp Pro
                325                 330                 335

Pro Ser Ala Thr Cys Pro Lys Gln Tyr Lys Arg Leu Gly Thr Leu Gln
            340                 345                 350

Ser Asn Phe Cys Ala Ser Ser Leu Val Val Thr Gly Thr Val Lys Thr
        355                 360                 365

Met Val Arg Gly Pro Gly Glu Gly Leu Thr Val Thr Ile Ser Leu Leu
    370                 375                 380

Gly Val Tyr Lys Ser Gly Gly Leu Asp Leu Pro Ser Pro Pro Thr Asp
385                 390                 395                 400

Thr Ser Leu Lys Leu Tyr Val Pro Cys Arg Gln Met Pro Pro Met Lys
                405                 410                 415

Lys Gly Ala Ser Tyr Leu Leu Met Gly Gln Val Glu Glu Asn Arg Gly
            420                 425                 430

Pro Ile Leu Pro Pro Glu Ser Phe Leu Val Pro Tyr Lys Pro Asn Gln
        435                 440                 445

Asp Gln Ile Leu Asn Asn Leu Arg Lys Arg Lys Cys Pro Ser Gln Pro
    450                 455                 460

Arg Pro Ala Ala
```

465

<210> 27
<211> 571
<212> PRT
<213> Unknown

<220>
<223> Sulfhydryl oxidase (Sulf)

<400> 27

```
Met Arg Arg Cys Gly Arg His Ser Gly Ser Pro Ser Gln Met Leu Leu
1               5                   10                  15

Leu Leu Leu Pro Pro Leu Leu Leu Ala Val Pro Gly Ala Gly Ala Val
            20                  25                  30

Gln Val Ser Val Leu Tyr Ser Ser Ser Asp Pro Val Thr Val Leu Asn
        35                  40                  45

Ala Asn Thr Val Arg Ser Thr Val Leu Arg Ser Asn Gly Ala Trp Ala
    50                  55                  60

Val Glu Phe Phe Ala Ser Trp Cys Gly His Cys Ile Ala Phe Ala Pro
65                  70                  75                  80

Thr Trp Lys Glu Leu Ala Tyr Asp Val Arg Glu Trp Arg Pro Val Leu
                85                  90                  95

Asn Leu Ala Val Leu Asp Cys Ala Glu Glu Thr Asn Thr Ala Val Cys
            100                 105                 110

Arg Asp Phe Asn Ile Ser Gly Phe Pro Thr Val Arg Phe Phe Lys Ala
        115                 120                 125

Phe Ser Lys Asn Gly Ser Gly Ile Thr Leu Pro Val Ala Asp Ala Ser
    130                 135                 140

Val Glu Thr Leu Arg Arg Lys Leu Ile Asp Ala Leu Glu Ser His Ser
145                 150                 155                 160

Asp Met Trp Ser Ser Ser Arg Pro Lys Leu Lys Pro Ala Lys Leu Val
                165                 170                 175

Glu Ile Asn Glu Phe Phe Ala Glu Thr Asn Glu Asp Tyr Leu Val Leu
            180                 185                 190

Ile Phe Glu Asp Lys Asp Ser Tyr Val Gly Arg Glu Val Thr Leu Asp
        195                 200                 205

Leu Phe Gln His His Ile Pro Val His Arg Val Leu Asn Thr Glu Arg
    210                 215                 220

Asn Ala Val Ser Lys Phe Gly Val Val Glu Phe Pro Ser Cys Tyr Leu
225                 230                 235                 240

Leu Phe Arg Asn Gly Ser Phe Ser Arg Val Pro Val Val Met Glu Ser
                245                 250                 255

Arg Leu Phe Tyr Thr Ser Tyr Leu Lys Gly Met Ser Gly Pro Ile Leu
            260                 265                 270
```

```
Val Asp Pro Pro Thr Thr Thr Ile Ser Thr Asp Ala Pro Val Thr Thr
        275                 280                 285

Asp Val Val Pro Thr Val Trp Lys Val Ala Asn His Ala Arg Ile Tyr
    290                 295                 300

Met Ala Asp Leu Glu Ser Ser Leu His Tyr Ile Phe Leu Val Glu Val
305                 310                 315                 320

Gly Lys Phe Ser Val Leu Glu Gly Gln Arg Leu Leu Ala Leu Lys Lys
                325                 330                 335

Leu Val Ala Val Leu Ala Lys Tyr Phe Pro Gly Arg Pro Leu Ala Gln
            340                 345                 350

Asn Phe Leu His Ser Ile His Asp Trp Leu Gln Arg Gln Gln Arg Lys
        355                 360                 365

Lys Ile Pro Tyr Lys Phe Phe Arg Ala Ala Leu Asp Asn Arg Lys Glu
    370                 375                 380

Gly Ile Val Leu Thr Glu Lys Val Asn Trp Val Gly Cys Gln Gly Ser
385                 390                 395                 400

Lys Pro His Phe Arg Gly Phe Pro Cys Ser Leu Trp Ile Leu Phe His
                405                 410                 415

Phe Leu Thr Val Gln Ala Ser Arg Tyr Ser Glu Asn His Pro Gln Glu
            420                 425                 430

Pro Ala Asp Gly Gln Glu Val Leu Gln Ala Met Arg Ser Tyr Val Gln
        435                 440                 445

Trp Phe Phe Gly Cys Arg Asp Cys Ala Glu His Phe Glu Asn Met Ala
    450                 455                 460

Ala Ser Thr Met His Arg Val Arg Ser Pro Thr Ser Ala Val Leu Trp
465                 470                 475                 480

Leu Trp Thr Ser His Asn Lys Val Asn Ala Arg Leu Ser Gly Ala Pro
                485                 490                 495

Ser Glu Asp Pro Tyr Phe Pro Lys Val Gln Trp Pro Leu Arg Glu Leu
            500                 505                 510

Cys Phe Asp Cys His Asn Glu Ile Asn Gly Arg Glu Pro Val Trp Asp
        515                 520                 525

Leu Glu Ala Thr Tyr Arg Phe Leu Lys Ala His Phe Ser Ser Glu Asn
    530                 535                 540

Ile Ile Leu Asp Thr Pro Val Ala Gly Leu Ala Thr Gln Arg Asn Pro
545                 550                 555                 560

Gln Ile Leu Gly Ala Thr Pro Glu Pro His Met
                565                 570
```

<210> 28
<211> 473
<212> PRT
<213> Unknown

<220>
<223> Lipoprotein lipase (Lip)

<400> 28

```
Met Glu Ser Lys Ala Leu Leu Leu Val Ala Leu Gly Val Trp Leu Gln
1               5                   10                  15

Ser Leu Thr Ala Ser Gln Gly Xaa Ala Ala Ala Asp Gly Gly Arg Asp
            20                  25                  30

Phe Thr Asp Ile Glu Ser Lys Phe Ala Leu Arg Thr Pro Asp Asp Thr
        35                  40                  45

Ala Glu Asp Asn Cys His Leu Ile Pro Gly Ile Ala Glu Ser Val Ser
    50                  55                  60

Asn Cys His Phe Asn His Ser Ser Lys Thr Phe Val Val Ile His Gly
65                  70                  75                  80

Trp Thr Val Thr Gly Met Tyr Glu Ser Trp Val Pro Lys Leu Val Ala
                85                  90                  95

Ala Leu Tyr Lys Arg Glu Pro Asp Ser Asn Val Ile Val Val Asp Trp
            100                 105                 110

Leu Tyr Arg Ala Gln Gln His Tyr Pro Val Ser Ala Gly Tyr Thr Lys
        115                 120                 125

Leu Val Gly Asn Asp Val Ala Arg Phe Ile Asn Trp Met Glu Glu Glu
    130                 135                 140

Phe Asn Tyr Pro Leu Asp Asn Val His Leu Leu Gly Tyr Ser Leu Gly
145                 150                 155                 160

Ala His Ala Ala Gly Val Ala Gly Ser Leu Thr Asn Lys Lys Val Asn
                165                 170                 175

Arg Ile Thr Gly Leu Asp Pro Ala Gly Pro Asn Phe Glu Tyr Ala Glu
            180                 185                 190

Ala Pro Ser Arg Leu Ser Pro Asp Asp Ala Asp Phe Val Asp Val Leu
        195                 200                 205

His Thr Phe Thr Arg Gly Ser Pro Gly Arg Ser Ile Gly Ile Gln Lys
    210                 215                 220

Pro Val Gly His Val Asp Ile Tyr Pro Asn Gly Gly Thr Phe Gln Pro
225                 230                 235                 240

Gly Cys Asn Ile Gly Glu Ala Ile Arg Val Ile Ala Glu Arg Gly Leu
                245                 250                 255

Gly Asp Val Asp Gln Leu Val Lys Cys Ser His Glu Arg Ser Ile His
            260                 265                 270

Leu Phe Ile Asp Ser Leu Leu Asn Glu Glu Asn Pro Ser Lys Ala Tyr
        275                 280                 285

Arg Cys Asn Ser Lys Glu Ala Phe Glu Lys Gly Leu Cys Leu Ser Cys
    290                 295                 300
```

Arg Lys Asn Arg Cys Asn Asn Val Gly Tyr Glu Ile Asn Lys Val Arg
305 310 315 320

Ala Lys Arg Ser Ser Lys Met Tyr Leu Lys Thr Arg Ser Gln Met Pro
325 330 335

Tyr Lys Val Phe His Tyr Gln Val Lys Ile His Phe Ser Gly Thr Glu
340 345 350

Ser Asp Lys Gln Leu Asn Gln Ala Phe Glu Ile Ser Leu Tyr Gly Thr
355 360 365

Val Ala Glu Ser Glu Asn Ile Pro Phe Thr Leu Pro Glu Val Ser Thr
370 375 380

Asn Lys Thr Tyr Ser Phe Leu Ile Tyr Thr Glu Val Asp Ile Gly Glu
385 390 395 400

Leu Leu Met Met Lys Leu Lys Trp Lys Ser Asp Ser Tyr Phe Ser Trp
405 410 415

Ser Asp Trp Trp Ser Ser Pro Gly Phe Val Ile Glu Lys Ile Arg Val
420 425 430

Lys Ala Gly Glu Thr Gln Lys Lys Val Ile Phe Cys Ala Arg Glu Lys
435 440 445

Val Ser His Leu Gln Lys Gly Lys Asp Ser Ala Val Phe Val Lys Cys
450 455 460

His Asp Lys Ser Leu Lys Lys Ser Gly
465 470

<210> 29
<211> 1212
<212> PRT
<213> Unknown

<220>
<223> Nidogen-1 (Nid)

<400> 29
Met Leu Asp Ala Ser Gly Trp Lys Pro Ala Ala Trp Thr Trp Val Leu
1 5 10 15

Leu Leu Gln Leu Leu Leu Ala Gly Pro Gly Asp Cys Leu Ser Arg Gln
20 25 30

Glu Leu Phe Pro Phe Gly Pro Gly Gln Gly Asp Leu Glu Leu Glu Ala
35 40 45

Gly Asp Asp Val Val Ser Pro Ala Leu Glu Leu Ile Gly Glu Leu Ser
50 55 60

Phe Tyr Asp Arg Ser Asp Ile Thr Ser Val Tyr Val Thr Thr Asn Gly
65 70 75 80

Ile Ile Ala Met Ser Glu Pro Pro Ala Arg Glu Ser His Pro Gly Thr
85 90 95

Phe Pro Pro Ser Phe Gly Ser Val Ala Pro Phe Leu Ala Asp Leu Asp

100 105 110

Thr Thr Asp Gly Leu Gly Asn Val Tyr Tyr Arg Glu Asp Leu Ser Pro
115 120 125

Ser Ile Met Gln Met Ala Ala Glu Tyr Val Gln Arg Gly Phe Pro Glu
130 135 140

Val Pro Phe Gln Pro Thr Ser Val Val Val Val Thr Trp Glu Ser Val
145 150 155 160

Ala Pro Tyr Glu Gly Pro Ser Gly Ser Ser Ala Gln Glu Gly Lys Arg
165 170 175

Asn Thr Phe Gln Ala Val Leu Ala Ser Ser Asn Ser Ser Ser Tyr Ala
180 185 190

Ile Phe Leu Tyr Pro Glu Asp Gly Leu Gln Phe Phe Thr Thr Phe Ser
195 200 205

Lys Lys Asp Glu Asn Gln Val Pro Ala Met Val Gly Phe Ser Gln Gly
210 215 220

Leu Val Gly Phe Leu Trp Arg Ser Asp Gly Ala Tyr Asn Ile Phe Ala
225 230 235 240

Asn Asp Arg Glu Ser Ile Glu Asn Leu Ala Lys Ser Ser Asn Ala Gly
245 250 255

His Gln Gly Val Trp Val Phe Glu Ile Gly Ser Pro Ala Thr Ala Lys
260 265 270

Gly Val Val Ser Ala Asp Val Asn Leu Gly Leu Asp Asp Asp Gly Ser
275 280 285

Asp Tyr Glu Asp Glu Glu Tyr Asp Leu Ala Thr Ser His Leu Gly Leu
290 295 300

Glu Asp Met Ala Thr Gln Pro Phe Pro Ser Pro Ser Pro Arg Arg Gly
305 310 315 320

Asn Thr His Pro His Asp Val Pro Arg Val Leu Ser Pro Ser Tyr Glu
325 330 335

Ala Thr Glu Arg Pro His Gly Ile Pro Thr Glu Arg Thr Arg Thr Phe
340 345 350

Gln Leu Pro Ala Glu Arg Phe His Gln Gln His Pro Gln Val Ile Asp
355 360 365

Val Asp Glu Val Glu Glu Thr Gly Ile Val Phe Ser Tyr Asn Ile Gly
370 375 380

Ser Gln Gln Thr Cys Ala Asn Asn Arg His Gln Cys Ser Val His Ala
385 390 395 400

Glu Cys Arg Asp Tyr Ala Thr Gly Phe Cys Cys Arg Cys Val Ala Asn
405 410 415

Tyr Thr Gly Asn Gly Arg Gln Cys Val Ala Glu Gly Ser Pro Gln Arg
420 425 430

Val Asn Gly Lys Val Lys Gly Arg Ile Phe Val Gly Asn Ser Gln Val

435 440 445

Pro Val Val Phe Glu Asn Thr Asp Leu His Ser Tyr Val Val Met Asn
450 455 460

His Gly Arg Ser Tyr Thr Ala Ile Ser Thr Ile Pro Glu Thr Val Gly
465 470 475 480

Tyr Ser Leu Leu Pro Leu Ala Pro Ile Gly Gly Ile Ile Gly Trp Met
485 490 495

Phe Ala Val Glu Gln Asn Gly Phe Lys Asn Gly Phe Ser Ile Thr Gly
500 505 510

Gly Glu Phe Thr Arg Gln Ala Glu Val Thr Phe Leu Gly His Pro Gly
515 520 525

Lys Leu Val Leu Lys Gln His Phe Ser Gly Ile Asp Glu His Gly His
530 535 540

Leu Thr Ile Asn Thr Glu Leu Asp Gly Arg Val Pro Gln Ile Pro Tyr
545 550 555 560

Gly Ser Ser Val His Ile Glu Pro Tyr Thr Glu Leu Tyr His Tyr Ser
565 570 575

Ser Ser Val Ile Thr Ser Ser Ser Thr Arg Glu Tyr Thr Val Thr Glu
580 585 590

Pro Asp Pro Asp Gly Thr Ala Pro Ser His Thr His Val Tyr Gln Trp
595 600 605

Arg Gln Thr Ile Thr Phe Gln Glu Cys Val His Asp Asp Ser Arg Pro
610 615 620

Ala Leu Pro Ser Thr Gln Gln Leu Ser Val Asp Ser Val Phe Val Leu
625 630 635 640

Tyr Asn Gln Glu Glu Arg Ile Leu Arg Tyr Ala Leu Ser Asn Ser Ile
645 650 655

Gly Pro Val Arg Glu Gly Ser Pro Asp Ala Leu Gln Asn Pro Cys Tyr
660 665 670

Ile Gly Thr His Gly Cys Asp Ser Asn Ala Ala Cys Arg Pro Gly Pro
675 680 685

Gly Thr Gln Phe Thr Cys Glu Cys Ser Ile Gly Phe Arg Gly Asp Gly
690 695 700

Gln Thr Cys Tyr Asp Ile Asp Glu Cys Ser Glu Gln Pro Ser Arg Cys
705 710 715 720

Gly Asn His Ala Ala Cys Asn Asn Ser Pro Gly Ala Tyr Leu Cys Glu
725 730 735

Cys Val Glu Gly Tyr His Phe Ser Asp Gly Gly Ile Cys Val Ala Asp
740 745 750

Val Asp Gln Arg Pro Ile Asn Tyr Cys Glu Thr Gly Leu His Asn Cys
755 760 765

Asp Ile Pro Gln Arg Ala Gln Cys Ile Tyr Met Gly Gly Ser Ser Tyr

770 775 780

Thr Cys Ser Cys Leu Pro Gly Phe Ser Gly Asp Gly Arg Ala Cys Gln
785 790 795 800

Asp Val Asp Glu Cys Gln Leu Ser Arg Cys His Pro Asp Ala Phe Cys
805 810 815

Tyr Asn Thr Pro Gly Ser Phe Thr Cys Gln Cys Lys Pro Gly Tyr Gln
820 825 830

Gly Asp Gly Phe Gln Cys Val Pro Gly Glu Val Gly Lys Thr Arg Cys
835 840 845

Gln Leu Glu Arg Glu His Ile Leu Gly Ala Ser Gly Val Ala Asp Ala
850 855 860

Gln Gln Pro Arg Leu Leu Gly Met Tyr Val Pro Gln Cys Asp Glu Tyr
865 870 875 880

Gly His Tyr Glu Pro Thr Gln Cys His His Gly Thr Gly Tyr Cys Trp
885 890 895

Cys Val Asp Arg Asp Gly Arg Glu Leu Glu Gly Thr Arg Thr Gln Pro
900 905 910

Gly Met Arg Pro Pro Cys Leu Ser Thr Val Ala Pro Pro Ile His Gln
915 920 925

Arg Pro Val Val Pro Thr Ala Val Ile Pro Leu Pro Pro Gly Thr His
930 935 940

Leu Leu Phe Ala Gln Thr Gly Lys Ile Glu Arg Leu Pro Leu Glu Gly
945 950 955 960

Asn Thr Met Lys Lys Thr Glu Ala Lys Ala Phe Phe His Ile Pro Ala
965 970 975

Lys Val Ile Ile Gly Leu Ala Phe Asp Cys Val Asp Lys Val Val Tyr
980 985 990

Trp Thr Asp Ile Ser Glu Pro Ser Ile Gly Arg Ala Ser Leu His Gly
995 1000 1005

Gly Glu Pro Thr Thr Ile Ile Arg Gln Asp Leu Gly Ser Pro Glu Gly
1010 1015 1020

Ile Ala Leu Asp His Leu Gly Arg Asn Ile Phe Trp Thr Asp Ser Gln
1025 1030 1035 1040

Leu Asp Arg Ile Glu Val Ala Arg Met Asp Gly Thr Gln Arg Arg Val
1045 1050 1055

Leu Phe Asp Thr Gly Leu Val Asn Pro Arg Gly Ile Val Thr Asp Ser
1060 1065 1070

Val Gly Gly Asn Leu Tyr Trp Thr Asp Trp Asn Arg Glu Asn Pro Lys
1075 1080 1085

Ile Glu Thr Ser Tyr Met Asp Gly Thr Asn Arg Arg Ile Leu Ala Gln
1090 1095 1100

Asp Asn Leu Gly Leu Pro Asn Gly Leu Thr Phe Asp Ala Phe Ser Ser

1105 1110 1115 1120

Gln Leu Cys Trp Val Asp Ala Gly Thr His Arg Ala Glu Cys Leu Asn
1125 1130 1135

Pro Ala Gln Pro Ser Ser Arg Lys Val Leu Glu Gly Leu Gln Tyr Pro
1140 1145 1150

Phe Ala Val Thr Ser Tyr Gly Lys Asn Leu Tyr Tyr Thr Asp Trp Lys
1155 1160 1165

Thr Asn Ser Val Ile Ala Met Asp Leu Ala Ile Ser Lys Glu Met Asp
1170 1175 1180

Ala Phe Thr Pro Thr Ser Arg Pro Gly Tyr Met Ala Ser Pro Leu Pro
1185 1190 1195 1200

Cys Pro Asn Ala Leu Lys Ala Thr Thr Thr Ala Gln
1205 1210

<210> 30
<211> 527
<212> PRT
<213> Unknown

<220>
<223> Protein disulfide-isomerase (Pro)

<400> 30
Met Asp Asp Arg Leu Leu Thr Val Leu Leu Leu Leu Leu Gly Val Ser
1 5 10 15

Gly Pro Trp Gly Gln Gly Gln Glu Pro Glu Gly Pro Ser Glu Val Leu
20 25 30

Pro Glu Glu Ser Ser Gly Glu Glu Val Pro Lys Glu Asp Gly Ile Leu
35 40 45

Val Leu Ser His His Thr Leu Ser Leu Ala Leu Gln Glu His Pro Ala
50 55 60

Leu Met Val Glu Phe Tyr Ala Pro Trp Cys Gly His Cys Lys Ala Leu
65 70 75 80

Ala Pro Glu Tyr Ser Lys Ala Ala Ala Leu Leu Ala Ala Glu Ser Ala
85 90 95

Ser Val Thr Leu Ala Lys Val Asp Gly Pro Ala Glu Pro Glu Leu Thr
100 105 110

Lys Glu Phe Gly Val Val Gly Tyr Pro Thr Leu Lys Phe Phe Gln Asn
115 120 125

Gly Asn Arg Thr Asn Pro Glu Glu Tyr Thr Gly Pro Gln Lys Ala Glu
130 135 140

Gly Ile Ala Glu Trp Leu Arg Arg Arg Val Gly Pro Ser Ala Lys Arg
145 150 155 160

Leu Glu Asp Glu Glu Asp Val Gln Ala Leu Thr Asp Lys Trp Glu Val
165 170 175

Val Val Ile Gly Phe Phe Gln Asp Leu Gln Gly Glu Asp Val Ala Thr
180 185 190

Phe Leu Ala Leu Ala Arg Asp Ala Leu Asp Ile Thr Phe Gly Phe Thr
195 200 205

Asp Gln Pro Gln Leu Phe Gln Lys Phe Gly Leu Thr Lys Asp Thr Val
210 215 220

Ile Leu Phe Lys Lys Phe Asp Glu Gly Arg Ala Asp Phe Pro Val Asp
225 230 235 240

Lys Asp Thr Gly Leu Asp Leu Gly Asp Leu Ser Arg Phe Leu Val Thr
245 250 255

His Ser Met His Leu Val Thr Glu Phe Asn Ser Gln Thr Ser Pro Lys
260 265 270

Ile Phe Ala Ala Lys Ile Leu Asn His Leu Leu Leu Phe Val Asn Lys
275 280 285

Thr Leu Ala Gln His Arg Glu Leu Leu Thr Asp Phe Arg Glu Ala Ala
290 295 300

Pro Pro Phe Arg Gly Gln Val Leu Phe Val Met Val Asp Val Ala Ala
305 310 315 320

Asp Asn Asp His Val Leu Asn Tyr Phe Gly Leu Lys Ala Glu Glu Ala
325 330 335

Pro Thr Leu Arg Leu Ile Asn Val Glu Thr Thr Lys Lys Tyr Ala Pro
340 345 350

Thr Gly Leu Val Pro Ile Thr Ala Ala Ser Val Ala Ala Phe Cys Gln
355 360 365

Ala Val Leu His Gly Gln Val Lys Pro Tyr Leu Leu Ser Gln Glu Ile
370 375 380

Pro Pro Asp Trp Asp Glu Arg Pro Val Lys Thr Leu Val Gly Lys Asn
385 390 395 400

Phe Glu Gln Val Ala Phe Asp Glu Thr Lys Asn Val Phe Val Lys Phe
405 410 415

Tyr Ala Pro Trp Cys Ser His Cys Lys Glu Met Ala Pro Ala Trp Glu
420 425 430

Ala Leu Ala Glu Lys Tyr Arg Asp Arg Glu Asp Ile Val Ile Ala Glu
435 440 445

Leu Asp Ala Thr Ala Asn Glu Leu Glu Ala Phe Ser Val His Gly Tyr
450 455 460

Pro Thr Leu Lys Phe Phe Pro Ala Gly Pro Asp Arg Lys Val Ile Glu
465 470 475 480

Tyr Lys Ser Thr Arg Asp Leu Glu Thr Phe Ser Lys Phe Leu Asp Ser
485 490 495

Gly Gly Asn Leu Pro Glu Glu Glu Pro Lys Glu Pro Ala Ile Ser Thr
500 505 510

Pro Glu Ile Gln Asp Asn Ser Thr Val Gly Pro Lys Glu Glu Leu
515 520 525

<210> 31
<211> 236
<212> PRT
<213> Unknown

<220>
<223> mCherry

<400> 31
Met Val Ser Lys Gly Glu Glu Asp Asn Met Ala Ile Ile Lys Glu Phe
1 5 10 15

Met Arg Phe Lys Val His Met Glu Gly Ser Val Asn Gly His Glu Phe
20 25 30

Glu Ile Glu Gly Glu Gly Glu Gly Arg Pro Tyr Glu Gly Thr Gln Thr
35 40 45

Ala Lys Leu Lys Val Thr Lys Gly Gly Pro Leu Pro Phe Ala Trp Asp
50 55 60

Ile Leu Ser Pro Gln Phe Met Tyr Gly Ser Lys Ala Tyr Val Lys His
65 70 75 80

Pro Ala Asp Ile Pro Asp Tyr Leu Lys Leu Ser Phe Pro Glu Gly Phe
85 90 95

Lys Trp Glu Arg Val Met Asn Phe Glu Asp Gly Gly Val Val Thr Val
100 105 110

Thr Gln Asp Ser Ser Leu Gln Asp Gly Glu Phe Ile Tyr Lys Val Lys
115 120 125

Leu Arg Gly Thr Asn Phe Pro Ser Asp Gly Pro Val Met Gln Lys Lys
130 135 140

Thr Met Gly Trp Glu Ala Ser Ser Glu Arg Met Tyr Pro Glu Asp Gly
145 150 155 160

Ala Leu Lys Gly Glu Ile Lys Gln Arg Leu Lys Leu Lys Asp Gly Gly
165 170 175

His Tyr Asp Ala Glu Val Lys Thr Thr Tyr Lys Ala Lys Lys Pro Val
180 185 190

Gln Leu Pro Gly Ala Tyr Asn Val Asn Ile Lys Leu Asp Ile Thr Ser
195 200 205

His Asn Glu Asp Tyr Thr Ile Val Glu Gln Tyr Glu Arg Ala Glu Gly
210 215 220

Arg His Ser Thr Gly Gly Met Asp Glu Leu Tyr Lys
225 230 235

<210> 32
<211> 711
<212> DNA
<213> Unknown

<220>
<223> gene of mCherry

<400> 32
atggtgagca agggcgagga ggataacatg gccatcatca aggagttcat gcgcttcaag 60

gtgcacatgg agggctccgt gaacggccac gagttcgaga tcgagggcga gggcgagggc 120

cgcccctacg agggcaccca gaccgccaag ctgaaggtga ccaagggtgg ccccctgccc 180

ttcgcctggg acatcctgtc ccctcagttc atgtacggct ccaaggccta cgtgaagcac 240

cccgccgaca tccccgacta cttgaagctg tccttccccg agggcttcaa gtgggagcgc 300

gtgatgaact tcgaggacgg cggcgtggtg accgtgaccc aggactcctc cctgcaggac 360

ggcgagttca tctacaaggt gaagctgcgc ggcaccaact tcccctccga cggccccgta 420

atgcagaaga agaccatggg ctgggaggcc tcctccgagc ggatgtaccc cgaggacggc 480

gccctgaagg gcgagatcaa gcagaggctg aagctgaagg acggcggcca ctacgacgct 540

gaggtcaaga ccacctacaa ggccaagaag cccgtgcagc tgcccggcgc ctacaacgtc 600

aacatcaagt tggacatcac ctcccacaac gaggactaca ccatcgtgga acagtacgaa 660

cgcgccgagg gccgccactc caccggcggc atggacgagc tgtacaagta a 711

<210> 33
<211> 22
<212> PRT
<213> Unknown

<220>
<223> SP7.2

<400> 33
Met His Arg Pro Glu Ala Met Leu Leu Leu Leu Thr Leu Ala Leu Leu
1 5 10 15

Gly Gly Pro Thr Trp Ala
20

<210> 34
<211> 66
<212> DNA
<213> Unknown

<220>
<223> gene of SP7.2

<400> 34
atgcaccggc cagaggccat gctgctgctg ctcacgcttg ccctcctggg gggccccacc 60

tgggca 66

<210> 35
<211> 88
<212> DNA
<213> Artificial Sequence

<220>
<223> Cat_SP_F

<400> 35
aggggtacc gccaccatgt ggtggtcctt gattccgctc tcttgcctgc tggcactggc 60

aagtgccgtg agcaagggcg aggaggat 88

<210> 36
<211> 54
<212> DNA
<213> Artificial Sequence

<220>
<223> CC_SP_F1

<400> 36
acagttgctg cctgtagcat ctatgtgctg gccgtgagca agggcgagga ggat 54

<210> 37
<211> 78
<212> DNA
<213> Artificial Sequence

<220>
<223> CC_SP_F2

<400> 37
aggggtacc gccaccatgc agttctccgc aagaacgctt ctgtgcctgc tactcacagt 60

tgctgcctgt agcatcta 78

<210> 38
<211> 52
<212> DNA
<213> Artificial Sequence

<220>
<223> Nuc_SP_F1

<400> 38
ggtcccgtgc atgcttactg ctctggaagc tgtgagcaag ggcgaggagg at 52

<210> 39
<211> 81
<212> DNA

<213> Artificial Sequence

<220>
<223> Nuc_SP_F2

<400> 39
agggggtacc gccaccatga ggtggaagat catccagcta cagtactgtt ttcttttggt 60

cccgtgcatg cttactgctc t 81

<210> 40
<211> 60
<212> DNA
<213> Artificial Sequence

<220>
<223> Clus_SP_F1

<400> 40
gggctgctgc tgacctggga caatggcatg gtcctgggag tgagcaaggg cgaggaggat 60

60

<210> 41
<211> 65
<212> DNA
<213> Artificial Sequence

<220>
<223> Clus_SP_F2

<400> 41
agggggtacc gccaccatga agattctcct gttgtgcgtg gggctgctgc tgacctggga 60

caatg 65

<210> 42
<211> 90
<212> DNA
<213> Artificial Sequence

<220>
<223> Pig_SP_F

<400> 42
ggtaccgcca ccatgcaggc cctggtgcta ctcctctgga caggagccct gcttgggcat 60

ggcagcagcg tgagcaaggg cgaggaggat 90

<210> 43
<211> 55
<212> DNA
<213> Artificial Sequence

<220>

<223> Proco_SP_F1

<400> 43
ccttgtggcc tgggtactgc ctcttgcccg aggcgtgagc aagggcgagg aggat 55

<210> 44
<211> 76
<212> DNA
<213> Artificial Sequence

<220>
<223> Proco_SP_F2

<400> 44
aggggtacc gccaccatgc tgcctgctgt cctaacctcc ctcctggggc cattccttgt 60

ggcctgggta ctgcct 76

<210> 45
<211> 75
<212> DNA
<213> Artificial Sequence

<220>
<223> Sulf_SP_F1

<400> 45
atgctactgc tgctgctgcc gccgctgctg ctcgcggtgc ccggcgctgg cgcggtgagc 60

aagggcgagg aggat 75

<210> 46
<211> 81
<212> DNA
<213> Artificial Sequence

<220>
<223> Sulf_SP_F2

<400> 46
aggggtacc gccaccatga ggaggtgcgg ccgccactcg ggtcgccgt cgcagatgct 60

actgctgctg ctgccgccgc t 81

<210> 47
<211> 58
<212> DNA
<213> Artificial Sequence

<220>
<223> Lip_SP_F1

<400> 47
ggtggctctg ggagtgtggc tccagagttt gaccgccgtg agcaagggcg aggaggat 58

<210> 48
<211> 62
<212> DNA
<213> Artificial Sequence

<220>
<223> Lip_SP_F2

<400> 48
aggggtacc gccaccatgg agagcaaagc cctgctcctg gtggctctgg gagtgtggct 60

cc 62

<210> 49
<211> 72
<212> DNA
<213> Artificial Sequence

<220>
<223> Nid_SP_F1

<400> 49
gggtgctgct gctgcagcta ttgctggcgg ggcccggaga ctgcctgagc cgtgagcaag 60

ggcgaggagg at 72

<210> 50
<211> 83
<212> DNA
<213> Artificial Sequence

<220>
<223> Nid_SP_F2

<400> 50
aggggtacc gccaccatgc tggacgcgag cggctggaag cccgcggcgt ggacatgggt 60

gctgctgctg cagctattgc tgg 83

<210> 51
<211> 65
<212> DNA
<213> Artificial Sequence

<220>
<223> Pro_SP_F1

<400> 51
tgttgctgct cctgctgggt gtctcaggcc catggggaca gggagtgagc aagggcgagg 60

aggat 65

<210> 52

<211> 65
<212> DNA
<213> Artificial Sequence

<220>
<223> Pro_SP_F2

<400> 52
agggggtacc gccaccatgg atgatcggct cctgacagtg ttgctgctcc tgctgggtgt 60

ctcag 65

<210> 53
<211> 36
<212> DNA
<213> Artificial Sequence

<220>
<223> mCherry XhoR

<400> 53
agggctcgag tttacttgta cagctcgtcc atgccg 36

<210> 54
<211> 36
<212> DNA
<213> Artificial Sequence

<220>
<223> Bgl_Hprt 5'F

<400> 54
agggagatct caccagtttt cattttctgt gtgaga 36

<210> 55
<211> 42
<212> DNA
<213> Artificial Sequence

<220>
<223> Hprt 5'_NruR

<400> 55
agggtcgcga attaggaata caaaataaat ctaggtcata gc 42

<210> 56
<211> 35
<212> DNA
<213> Artificial Sequence

<220>
<223> Sal_Hprt 3'F

<400> 56

aggggtcgac cactatgtcg aggatttgga aaagg 35

<210> 57
<211> 34
<212> DNA
<213> Artificial Sequence

<220>
<223> Hprt 3'_SalR

<400> 57
aggggtcgac gaggatggga gtgagagtga actg 34

<210> 58
<211> 218
<212> PRT
<213> Unknown

<220>
<223> Light chain of Pembrolizumab

<400> 58
```
Glu Ile Val Leu Thr Gln Ser Pro Ala Thr Leu Ser Leu Ser Pro Gly
1               5                   10                  15

Glu Arg Ala Thr Leu Ser Cys Arg Ala Ser Lys Gly Val Ser Thr Ser
            20                  25                  30

Gly Tyr Ser Tyr Leu His Trp Tyr Gln Gln Lys Pro Gly Gln Ala Pro
        35                  40                  45

Arg Leu Leu Ile Tyr Leu Ala Ser Tyr Leu Glu Ser Gly Val Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Val Tyr Tyr Cys Gln His Ser Arg
                85                  90                  95

Asp Leu Pro Leu Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
```

195 200 205

Val Thr Lys Ser Phe Asn Arg Gly Glu Cys
210 215

<210> 59
<211> 447
<212> PRT
<213> Unknown

<220>
<223> Heavy chain of Pembrolizumab

<400> 59
Gln Val Gln Leu Val Gln Ser Gly Val Glu Val Lys Lys Pro Gly Ala
1 5 10 15

Ser Val Lys Val Ser Cys Lys Ala Ser Gly Tyr Thr Phe Thr Asn Tyr
20 25 30

Tyr Met Tyr Trp Val Arg Gln Ala Pro Gly Gln Gly Leu Glu Trp Met
35 40 45

Gly Gly Ile Asn Pro Ser Asn Gly Gly Thr Asn Phe Asn Glu Lys Phe
50 55 60

Lys Asn Arg Val Thr Leu Thr Thr Asp Ser Ser Thr Thr Thr Ala Tyr
65 70 75 80

Met Glu Leu Lys Ser Leu Gln Phe Asp Asp Thr Ala Val Tyr Tyr Cys
85 90 95

Ala Arg Arg Asp Tyr Arg Phe Asp Met Gly Phe Asp Tyr Trp Gly Gln
100 105 110

Gly Thr Thr Val Thr Val Ser Ser Ala Ser Thr Lys Gly Pro Ser Val
115 120 125

Phe Pro Leu Ala Pro Cys Ser Arg Ser Thr Ser Glu Ser Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Lys Thr Tyr Thr Cys Asn Val Asp His Lys
195 200 205

Pro Ser Asn Thr Lys Val Asp Lys Arg Val Glu Ser Lys Tyr Gly Pro
210 215 220

Pro Cys Pro Pro Cys Pro Ala Pro Glu Phe Leu Gly Gly Pro Ser Val
225 230 235 240

Phe Leu Phe Pro Pro Lys Pro Lys Asp Thr Leu Met Ile Ser Arg Thr
245 250 255

```
Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Arg Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys
        435                 440                 445
```